(19) Europäisches Patentamt
European Patent Office
Office européen des brevets

(11) **EP 4 136 258 B1**

(12) **EUROPEAN PATENT SPECIFICATION**

(45) Date of publication and mention
of the grant of the patent:
**04.12.2024 Bulletin 2024/49**

(21) Application number: **21718579.2**

(22) Date of filing: **15.04.2021**

(51) International Patent Classification (IPC):
*C12Q 1/6851* *(2018.01)*

(52) Cooperative Patent Classification (CPC):
(C-Sets available)
**C12Q 1/6851** (Cont.)

(86) International application number:
**PCT/EP2021/059836**

(87) International publication number:
**WO 2021/209573 (21.10.2021 Gazette 2021/42)**

(54) **METHOD FOR DETECTING GENETIC EVENTS**

GEPOOLTE TESTS VON INFEKTIONSKRANKHEITEN ODER GENETISCHEN EREIGNISSEN

DÉPISTAGE GROUPÉ DES MALADIES INFECTIEUSES OU DES ÉVÉNEMENTS GÉNÉTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.04.2020 FR 2003789
01.06.2020 EP 20177695**

(43) Date of publication of application:
**22.02.2023 Bulletin 2023/08**

(73) Proprietor: **Stilla Technologies
94800 Villejuif (FR)**

(72) Inventors:
• **ANDRÉ, Barbara
75012 Paris (FR)**
• **MALLORY, Allison
94800 Villejuif (FR)**
• **DANGLA, Rémi
75009 Paris (FR)**
• **FERNANDEZ, Nicolas
75012 Paris (FR)**

(74) Representative: **IPAZ
Bâtiment Platon
Parc Les Algorithmes
91190 Saint-Aubin (FR)**

(56) References cited:
**WO-A1-2016/003047**

• **FAN H C ET AL: "Microfluidic digital PCR enables
rapid prenatal diagnosis of fetal aneuploidy",
AMERICAN JOURNAL OF OBSTETRICS &
GYNECOLOGY, MOSBY, ST LOUIS, MO, US, vol.
200, no. 5, 1 May 2009 (2009-05-01), pages 543.e1
- 543.e7, XP026056021, ISSN: 0002-9378,
[retrieved on 20090416], DOI:
10.1016/J.AJOG.2009.03.002**
• **TAN CHIANRU ET AL: "A multiplex droplet digital
PCR assay for non-invasive prenatal testing of
fetal aneuploidies", ANALYST, vol. 144, no. 7, 8
January 2019 (2019-01-08), UK, pages 2239 - 2247,
XP055820640, ISSN: 0003-2654, DOI:
10.1039/C8AN02018C**
• **EMMANUEL DEBRAND ET AL: "A Non-Invasive
Droplet Digital PCR (ddPCR) Assay to Detect
Paternal CFTR Mutations in the Cell-Free Fetal
DNA (cffDNA) of Three Pregnancies at Risk of
Cystic Fibrosis via Compound Heterozygosity",
PLOS ONE, vol. 10, no. 11, 11 November 2015
(2015-11-11), pages e0142729, XP055445510,
DOI: 10.1371/journal.pone.0142729**
• **TAKESHITA TAKASHI ET AL: "Droplet digital
polymerase chain reaction assay for screening
of ESR1mutations in 325 breast cancer
specimens", TRANSLATIONAL RESEARCH, vol.
166, no. 6, 1 December 2015 (2015-12-01), pages
540, XP029304641, ISSN: 1931-5244, DOI:
10.1016/J.TRSL.2015.09.003**

- **SUN KUN ET AL: "The impact of digital DNA counting technologies on noninvasive prenatal testing", EXPERT REVIEW OF MOLECULAR DIAGNOSTICS, EXPERT REVIEWS LTD, GB, vol. 15, no. 10, 1 January 2015 (2015-01-01), pages 1261 - 1268, XP009187658, ISSN: 1744-8352, DOI: 10.1586/14737159.2015.1084227**
- **TONG YU ET AL: "Application of Digital PCR in Detecting Human Diseases Associated Gene Mutation", CELLULAR PHYSIOLOGY AND BIOCHEMISTRY, vol. 43, no. 4, 1 November 2017 (2017-11-01), CH, pages 1718 - 1730, XP055820674, ISSN: 1015-8987, Retrieved from the Internet <URL:https://www.karger.com/Article/Pdf/48403 5> DOI: 10.1159/000484035**

(52) Cooperative Patent Classification (CPC): (Cont.)

C-Sets
**C12Q 1/6851, C12Q 2521/301, C12Q 2563/159, C12Q 2565/102**

**Description**

**FIELD OF INVENTION**

The present invention relates to a method of detecting a genetic event, the method comprising steps of partitioning a sample from a subject into a plurality of partitions, and carrying out a digital polymerase chain reaction (dPCR) assay, to determine occurrence of said genetic event.

**BACKGROUND OF INVENTION**

It is known to perform prenatal testing during pregnancy to detect problems that may affect the embryo or the fetus. Such problems may include chromosome abnormalities and gene mutations that are likely to cause genetic disorders and birth defects.

Known prenatal tests include chorionic villus sampling, amniocentesis, percutaneous umbilical cord blood sampling, and the like. These tests have been proven to allow detection of the aforementioned problems.

For instance, document Tan Chianru et al.: "A multiplex droplet digital PCR assay for non-invasive prenatal testing of fetal aneuploidies", ANALYST, vol. 144, no. 7, 8 January 2019 (2019-01-08), pages 2239-2247, discloses a method of determining fetal aneuploidies by ddPCR.

However, these tests are not satisfactory.

Indeed, such tests are invasive, so that they are constraining, induce discomfort during their implementation, and may be associated to an increased risk of miscarriage.

A purpose of the invention is to provide a method for detecting a genetic event that is safer and less constraining than known methods, while being quick, effective and reliable.

**SUMMARY**

The present invention relates to a method according to claim 1.

The claimed method allows to perform noninvasive prenatal tests that are quick, effective and accurate, that is, reliable. This is due to the fact that nucleic acids from an embryo or a fetus can be found in samples of the pregnant woman carrying said embryo or fetus, such as in her blood.

Even though these nucleic acids are present in very small quantities in said samples, dPCR provides an increased sensitivity, allowing detection of said nucleic acids.

Furthermore, using several nucleic acid probes attached to detectable labels that have non-overlapping excitation wavelength ranges and/or non-overlapping emission wavelength ranges, the nucleic acid fragments that are sought are detected through separate detection channels of the system preforming the dPCR assay. As a result, both the limit of detection and the measurement uncertainty are significantly reduced, which enhances the sensitivity of the test.

As a result, even though these nucleic acids of an embryo or a fetus are present in very small quantities in said samples of the pregnant woman carrying said embryo or fetus, a prenatal test implementing the detection method as claimed is effective and reliable, while inducing minimal discomfort.

According to other advantageous aspects of the invention, the method includes one or more of the features of the dependent claims, taken either alone or in any possible combination.

The invention also relates to a non-invasive prenatal testing method according to claim 11.

The present invention also relates to a system according to claim 12.

**DEFINITIONS**

In the present disclosure, the following terms have the following meanings:

The term **"about",** as used herein before a figure or value, refers to a margin of error in said figure or value that the one skilled in the art can readily appreciate. In particular, the term "about" may refer to a margin of error of 1%, 2%, 5% or 10%.

The term **"amplicon"** refers to a product of an amplification reaction. An amplicon may be single-stranded or double-stranded, or a combination thereof. An amplicon corresponds to any suitable segment or the entire length of a nucleic acid target.

The term **"amplification"** refers to a reaction in which replication occurs repeatedly over time to form multiple copies of at least one segment of a template molecule. Amplification may generate an exponential or linear increase in the number of copies as amplification proceeds. Typical amplifications produce a greater than 1,000-fold increase in copy number and/or signal. Exemplary amplification reactions for the droplet-based assays disclosed herein may include the polymerase chain reaction (PCR) or ligase chain reaction, each of which is driven by thermal cycling. The droplet-based assays also or alternatively may use other amplification reactions, which may be performed isothermally, such as branched-probe DNA assays, cascade rolling circle amplification (cascade-RCA), helicase-dependent amplification, loop-mediated isothermal amplification (LAMP), nucleic acid based amplification (NASBA), nicking enzyme amplification reaction (NEAR), PAN-AC, Q-beta replicase amplification, rolling circle amplification (RCA), self-sustaining sequence replication, strand-displacement amplification, and the like. Amplification may utilize a linear or circular template. Amplification may be performed with any suitable reagents. Amplification may be performed, or assayed for its occurrence, in an amplification mixture, which is any composition capable of generating multiple copies of a nucleic acid target molecule, if present, in the composition. An **"amplification mixture"** may include any combination of at least one primer or primer pair, at least one reporter (such as, e.g., a nucleic acid probe and/or a dye), at least one replication enzyme (e.g., at least one polymerase, such as at least one DNA and/or RNA polymerase), and deoxynucleotide (and/or nucleotide) triphosphates (dNTPs and/or NTPs), among others.

The term **"analyte"** refers to a component(s) or potential component(s) of a sample that is analyzed in an assay. An **"analyte"** is a specific subject of interest in an assay where the **"sample"** is the general subject of interest. An analyte may, for example, be a nucleic acid, protein, peptide, enzyme, cell, bacteria, spore, virus, organelle, macromolecular assembly, drug candidate, lipid, carbohydrate, metabolite, or any combination thereof, among others. An analyte may be assayed for its presence, activity and/or other characteristic in a sample and/or in partitions thereof. The presence of an analyte may relate to an absolute or relative number, concentration, binary assessment (e.g., present or absent), or the like, of the analyte in a sample or in one or more partitions thereof. In some examples, a sample may be partitioned such that a copy of the analyte is not present in all of the partitions, such as being present in the partitions at an average concentration of about 0.0001 to 10000, 0.001 to 1000, 0.01 to 100, 0.1 to 10, or one copy per partition.

The term **"assay"** refers to a procedure(s) and/or reaction(s) used to characterize a sample, and any signal(s), value(s), data, and/or result(s) obtained from the procedure(s) and/or reaction(s). Exemplary droplet-based assays are biochemical assays using aqueous assay mixtures. More particularly, the droplet-based assays may be enzyme assays and/or binding assays, among others. The enzyme assays may, for example, determine whether individual droplets contain a copy of a substrate molecule *(e.g.,* a nucleic acid target) for an enzyme and/or a copy of an enzyme molecule. Based on these assay results, a concentration and/or copy number of the substrate and/or the enzyme in a sample may be estimated.

The term **"digital PCR"** or **"dPCR"** refers to a PCR assay performed on portions of a sample to determine the presence/absence, concentration, and/or copy number of a nucleic acid target in the sample, based on how many of the sample portions support amplification of the target. Digital PCR may (or may not) be performed as endpoint PCR. Digital PCR may (or may not) be performed as real-time PCR for each of the partitions. PCR theoretically results in an exponential amplification of a nucleic acid sequence (analyte) from a sample. By measuring the number of amplification cycles required to achieve a threshold level of amplification (as in real-time PCR), one can theoretically calculate the starting concentration of nucleic acid. In practice, however, there are many factors that make the PCR process non-exponential, such as varying amplification efficiencies, low copy numbers of starting nucleic acid, and competition with background contaminant nucleic acid. Digital PCR is generally insensitive to these factors, since it does not rely on the assumption that the PCR process is exponential. In digital PCR, individual nucleic acid molecules are separated from the initial sample into partitions, then amplified to detectable levels. Each partition then provides digital information on the presence or absence of each individual nucleic acid molecule within each partition. When enough partitions are measured using this technique, the digital information can be consolidated to make a statistically relevant measure of starting concentration for the nucleic acid target (analyte) in the sample. The concept of digital PCR may be extended to other types of analytes, besides nucleic acids. In particular, a signal amplification reaction may be utilized to permit detection of a single copy of a molecule of the analyte in individual droplets, to permit data analysis of droplet signals for other analytes (e.g., using an algorithm based on Poisson statistics). Exemplary signal amplification reactions that permit detection of single copies of other types of analytes in droplets include enzyme reactions.

The term **"droplet"** refers to a small volume of liquid (such as a dispersed phase), typically with a spherical shape, encapsulated by an immiscible fluid (such as a continuous phase). The volume of a droplet and/or the average volume of a population of droplets, may, e.g., be less than about 1 μL (and is therefore termed **"microdroplet"),** less than about 1 nL, or less than about 1 pL. A droplet (or a population of droplets) may have a diameter (or an average diameter) of less than about 1000 μm, about 100 μm, about 10 μm; or ranging from about 10 μm to about 1000 μm. A droplet may be spherical or non-spherical. A droplet may be a simple droplet or a compound droplet *(i.e.,* a droplet encapsulating at least one droplet). The droplets of an emulsion may have any uniform or non-uniform distribution in the continuous phase. If non-uniform, the concentration of the droplets may vary to provide one or more regions of higher droplet density and one or more regions of lower droplet density in the continuous phase. For example, droplets may sink or float in the continuous phase, may be clustered in one or more packets along a channel or in a storage chamber, may be focused toward the center or perimeter of a flow stream, or the like. In some embodiments of the present disclosure, a droplet has a diameter (or an average diameter) ranging from about 10 μm to about 150 μm, preferably from about 25 μm to about 125 μm, more preferably from about 50 μm to about 100 μm, even more preferably from

about 65 μm to about 80 μm. In some embodiments of the present disclosure, a droplet has a diameter (or an average diameter) of about 10 μm ±5 μm, 20 μm ±5 μm, 30 μm ±5 μm, 40 μm ± 5 μm, 50 μm ± 5 μm, 60 μm ± 5 μm, 70 μm ± 5 μm, 80 μm ± 5 μm, 90 μm ± 5 μm, 100 μm ± 5 μm, 110 μm ± 5 μm, 120 μm ± 5 μm, 130 μm ± 5 μm, 140 μm ± 5 μm, 150 μm ± 5 μm.

The diameter of a droplet can also be mathematically defined as a function of its volume, with the following formula:

$$diameter = \sqrt[3]{volume \times \frac{6}{\pi}}$$

In some embodiments of the present disclosure, a droplet has a volume (or an average volume) ranging from about 1 pL to about 1 nL, preferably from about 50 pL to about 750 pL, more preferably from about 100 pL to about 500 pL, even more preferably from about 150 pL to about 250 pL. In some embodiments of the present disclosure, a droplet has a volume (or an average volume) of 1 pL, 10 pL, 25 pL, 50 pL, 75 pL, 100 pL, 125 pL, 150 pL, 175 pL, 200 pL, 225 pL, 250 pL, 275 pL, 300 pL, 400 pL, 500 pL, 600 pL, 700 pL, 800 pL, 900 pL, 1 nL. It will be readily understood by the one skilled in the art that such diameters and/or volumes are subject to a fair margin of error.

The term **"endpoint PCR"** refers to a PCR-based analysis in which amplicon formation is measured after the completion of thermal cycling.

The term **"label"** refers to an identifying and/or distinguishing marker or identifier connected to or incorporated into any entity, such as a compound, biological particle (e.g., a cell, bacteria, spore, virus, or organelle), or droplet. A label may, for example, be a dye that renders an entity optically detectable and/or optically distinguishable. Exemplary dyes used for labeling are fluorescent dyes (fluorophores) and fluorescence quenchers.

The term **"microfluidic chip"** refers to a substrate containing microfluidic channels, wherein volumes down to picoliters (pL) are handled within the microfluidic channels of the microfluidic chip. A wide variety of methods and materials exists and will be known and appreciated by the one skilled in the art for construction of microfluidic channels and networks thereof. For example, the microfluidic channel may be constructed using simple tubing, but may further involve sealing the surface of one slab comprising etched open channels to a second flat slab. Materials into which microfluidic channels may be formed include silicon, glass, polydimethylsiloxane (PDMS), and plastics (such as polymethylmethacrylate, cyclic olefin polymer [COP], cyclic olefin copolymer [COC], polypropylene, among others). The same materials can also be used for the second sealing slab. Compatible combinations of materials for the two slabs depend on the method employed to seal them together. The microfluidic channel may be encased as desired in an optically clear material to allow for optical excitation (resulting in, e.g., fluorescence) or illumination (resulting in, e.g., selective absorption) of a sample as desired, and to allow for optical detection of spectroscopic properties of light from a sample in the microfluidic chip. Preferred examples of such optically clear materials that exhibit high optical clarity and low autofluorescence include, but are not limited to, borosilicate glass (e.g., SCHOTT BOROFLOAT® glass [Schott North America, Elmsford NY]) and cyclo-olefin polymers (COP) (e.g., ZEONOR® [Zeon Chemicals LP, Louisville KY]).

The term **"nucleic acid"** refers to both DNA or RNA, whether it be a product of amplification, synthetically created, products of reverse transcription of RNA or naturally occurring. Typically, nucleic acids are single- or double-stranded molecules and are composed of naturally occurring nucleotides. Double-stranded nucleic acid molecules can have 3' or 5' overhangs and as such are not required or assumed to be completely double-stranded over their entire length. Furthermore, the term nucleic acid can be composed of non-naturally occurring nucleotides and/or modifications to naturally occurring nucleotides. Examples are listed herein, but are not limited to, phosphorylation of 5' or 3' nucleotides to allow for ligation or prevention of exonuclease degradation/polymerase extension, respectively; amino, thiol, alkyne, or biotinyl modifications for covalent and near covalent attachments; fluorophores and quenchers; phosphorothioate, methylphosphonates, phosphoroamidates and phosphorotiester linkages between nucleotides to prevent degradation; methylation; and modified bases such as deoxyinosine, 5-bromo dU, deoxyuridine, 2-aminopurine, dideoxycytidine, 5-methyl dC, locked nucleic acids (LNA's), iso-dC and -dG bases, 2'-O-methyl RNA bases and fluorine modified bases.

The term **"nucleotide"** in addition to referring to the naturally occurring ribonucleotide or deoxyribonucleotide monomers, shall herein be understood to refer to related structural variants thereof, including derivatives and analogs, that are functionally equivalent with respect to the particular context in which the nucleotide is being used (e.g., hybridization to a complementary base), unless the context clearly indicates otherwise.

The term **"partition"** refers to a separated portion of a bulk volume. The partition may be a sample partition generated from a sample, such as a prepared sample, that forms the bulk volume. Partitions generated from a bulk volume may be substantially uniform in size or may have distinct sizes (*e.g.*, sets of partitions of two or more discrete, uniform sizes). Exemplary partitions are **"droplets"**. Partitions may also vary in size with a predetermined size distribution or with a random size distribution.

The term **"PCR"** or **"polymerase chain reaction"** refers to a nucleic acid amplification assay that relies on alternating cycles of heating and cooling (*i.e.,* thermal cycling) to achieve successive rounds of replication. PCR may be performed by thermal cycling between two or more temperature set points, such as a higher melting (denaturation) temperature and a lower annealing/extension temperature, or among three or more temperature set points, such as a higher melting temperature, a lower annealing temperature, and an intermediate extension temperature, among others. PCR may be performed with a thermostable polymerase, such as Taq DNA polymerase (e.g., wild-type enzyme, a Stoffel fragment, FastStart polymerase, etc.), Pfu DNA polymerase, S-Tbr polymerase, Tth polymerase, Vent polymerase, or a combination thereof, among others. PCR generally produces an exponential increase in the amount of a product amplicon over successive cycles. Any suitable PCR methodology or combination of methodologies may be utilized in the droplet-based assays disclosed herein, such as allele-specific PCR, assembly PCR, asymmetric PCR, digital PCR, endpoint PCR, hot-start PCR, in situ PCR, intersequence-specific PCR, inverse PCR, linear after exponential PCR, ligation-mediated PCR, methylation-specific PCR, miniprimer PCR, multiplex ligation-dependent probe amplification, multiplex PCR, nested PCR, overlap-extension PCR, polymerase cycling assembly, qualitative PCR, quantitative PCR, real-time PCR, RT-PCR, single-cell PCR, solid-phase PCR, thermal asymmetric interlaced PCR, touchdown PCR, or universal fast walking PCR, among others.

The term **"primer"** refers to a polynucleotide capable of acting as a point of initiation of template-directed nucleic acid synthesis when placed under conditions in which polynucleotide extension is initiated (*e.g.*, under conditions comprising the presence of requisite nucleoside triphosphates (as dictated by the template that is copied) and a polymerase in an appropriate buffer and at a suitable temperature or cycle(s) of temperatures (*e.g.*, as in a polymerase chain reaction)). To further illustrate, primers can also be used in a variety of other oligonucleotide-mediated synthesis processes, including as initiators of de novo RNA synthesis and in vitro transcription-related processes (*e.g.*, nucleic acid sequence-based amplification (NASBA), transcription mediated amplification (TMA), etc.). A primer is typically a single-stranded oligonucleotide (*e.g.*, oligodeoxyribonucleotide). The appropriate length of a primer depends on the intended use of the primer but typically ranges from 6 to 40 nucleotides, more typically from 15 to 35 nucleotides. Short primer molecules generally require cooler temperatures to form sufficiently stable hybrid complexes with the template. A primer need not reflect the exact sequence of the template but is usefully sufficiently complementary to hybridize with a template for primer elongation to occur. In certain embodiments, the term **"primer pair"** means a set of primers including a 5' sense primer (sometimes called **"forward"**) that hybridizes with the complement of the 5' end of the target sequence to be amplified and a 3' antisense primer (sometimes called **"reverse"**) that hybridizes with the 3' end of the target sequence to be amplified (*e.g.*, if the target sequence is expressed as RNA or is an RNA). A primer can be labeled, if desired, by incorporating a label detectable by spectroscopic, photochemical, biochemical, immunochemical, or chemical means. For example, useful labels include 32P, fluorescent dyes, electron-dense reagents, enzymes (as commonly used in ELISA assays), biotin or haptens and proteins for which antisera or monoclonal antibodies are available.

The term **"probe"** refers to a nucleic acid attached or linked to at least one detectable label, such as at least one dye. A probe may be a sequence-specific binding partner for a nucleic acid target and/or amplicon. The probe may be designed to enable detection of target amplification based on fluorescence resonance energy transfer (FRET). An exemplary probe for the nucleic acid assays disclosed herein includes one or more nucleic acids attached or linked to a pair of dyes that collectively exhibit fluorescence resonance energy transfer (FRET) when proximate one another. The pair of dyes may provide first and second emitters, or an emitter and a quencher, among others. Fluorescence emission from the pair of dyes changes when the dyes are separated from one another, such as by cleavage of the probe during primer extension (*e.g.*, a 5' nuclease assay, such as with a TaqMan™ probe), or when the probe hybridizes to an amplicon (*e.g.*, a molecular beacon probe). The nucleic acid portion of the probe may have any suitable structure or origin, for example, the portion may be a locked nucleic acid (LNA), a member of a universal probe library, or the like. In other cases, a probe and one of the primers of a primer pair may be combined in the same molecule (e.g., Amplifluor™ primers or Scorpions® primers). As an example, the primer-probe molecule may include a primer sequence at its 3' end and a molecular beacon-style probe at its 5' end. With this arrangement, related primer-probe molecules labeled with different dyes can be used in a multiplexed assay with the same reverse primer to quantify target sequences differing by a single nucleotide (single nucleotide polymorphisms (SNPs)). Another exemplary probe for droplet-based nucleic acid assays is a Plexor primer.

The term **"reagent"** refers to a compound, set of compounds, and/or composition that is combined with a sample in order to perform a particular assay(s) on the sample. A reagent may be a target-specific reagent, which is any reagent composition that confers specificity for detection of a particular target(s) or analyte(s) in an assay. A reagent optionally may include a chemical reactant and/or a binding partner for the assay. A reagent may, for example, include at least one nucleic acid, protein (*e.g.,* an enzyme), cell, virus, organelle, macromolecular assembly, potential drug, lipid, carbohydrate, inorganic substance, or any combination thereof, and may be an aqueous composition, among others. In exemplary embodiments, the reagent may be an amplification reagent, which may include at least one primer or at least one pair of primers for amplification of a nucleic acid target, at least one a reporter (such as, e.g., a nucleic acid probe and/or a dye) to enable detection of amplification, a polymerase, nucleotides (dNTPs and/or NTPs), divalent magnesium ions, potassium chloride, buffer, or any combination thereof, among others.

The term **"replication"** refers to a process forming a copy *(i.e.,* a direct copy and/or a complimentary copy) of a nucleic acid or a segment thereof. Replication generally involves an enzyme, such as a polymerase and/or a ligase, among others. The nucleic acid and/or segment replicated is a template (and/or a target) for replication.

The term **"reporter"** refers to a compound or set of compounds that reports a condition, such as the extent of a reaction. Exemplary reporters comprise at least one dye, such as a fluorescent dye or an energy transfer pair, and/or at least one oligonucleotide. Exemplary reporters for nucleic acid amplification assays may include a nucleic acid probe comprising a detectable label and/or an intercalating dye (e.g., SYBR Green, ethidium bromide, etc.).

The terms **"reverse transcription PCR"** or **"RT-PCR"** refer to a PCR assay utilizing a complementary DNA template produced by reverse transcription of RNA. RT-PCR permits analysis of an RNA sample by (1) forming complementary DNA **("cDNA")** copies of RNA, such as with a reverse transcriptase enzyme, and (2) PCR amplification using the cDNA as a template. The reverse-transcription of RNAs may be carried out by techniques well known to the skilled artisan, using a reverse transcriptase enzyme and a mix of 4 deoxyribonucleotides triphosphate (dNTPs), namely deoxyadenosine triphosphate (dATP), deoxycytidine triphosphate (dCTP), deoxyguanosine triphosphate (dGTP) and (deoxy)thymidine triphosphate (dTTP). In some embodiments, the reverse-transcription of RNAs comprises a first step of first-strand cDNA synthesis. Methods for first-strand cDNA synthesis are well-known to the skilled artisan. First-strand cDNA synthesis reactions can use a combination of sequence-specific primers, oligo(dT) primers or random primers. Examples of reverse transcriptase enzymes include, but are not limited to, M-MLV reverse transcriptase, SuperScript II (Invitrogen), SuperScript III (Invitrogen), SuperScript IV (Invitrogen), Maxima (ThermoFisher Scientific), ProtoScript II (New England Biolabs), PrimeScript (ClonTech).

The term **"sample"** refers to a compound, composition, and/or mixture of interest, from any suitable source(s). A sample is the general subject of interest for an assay that analyzes an aspect of the sample, such as an aspect related to at least one analyte that may be present in the sample. Samples may be analyzed in their natural state, as collected, and/or in an altered state, for example, following storage, preservation, extraction, lysis, dilution, concentration, purification, filtration, mixing with one or more reagents, pre-amplification (e.g., to achieve target enrichment by performing limited cycles *(e.g.,* < 15) of PCR on sample prior to PCR), removal of amplicon (e.g., treatment with uracil-d-glycosylase (UDG) prior to PCR to eliminate any carry-over contamination by a previously generated amplicon (*i.e.*, the amplicon is digestable with UDG because it is generated with dUTP instead of dTTP)), partitioning, or any combination thereof, among others. In one embodiment, the sample is a **"clinical sample".** Clinical samples may include nasopharyngeal wash, blood, plasma, cell-free plasma, buffy coat, saliva, urine, stool, sputum, mucous, wound swab, tissue biopsy, milk, a fluid aspirate, a swab (*e.g.*, a nasopharyngeal swab), and/or tissue from a subject, among others. In some embodiments, the sample may comprise one or several reagents, such as, *e.g.*, an amplification mixture.

## DETAILED DESCRIPTION

The present invention relates to a method of detecting a genetic event. More precisely, the invention relates to a method for detecting the occurrence of a genetic event in a subj ect.

As used herein, the term **"genetic event"** is used to define any mutation in a nucleic acid sequence of a subject or any chromosome abnormality in a subject. Examples of mutations include, but are not limited to, insertions, deletion, substitutions, transversions, transitions, translocations, inversions, and the like. Examples of chromosome abnormalities include, but are not limited to, monosomies, trisomies, tetrasomies, and the like; such as, e.g., Down syndrome 21, Edwards syndrome 18, Patau syndrome 13, trisomy 9, tetrasomy 9p, Warkany syndrome 2, cat eye syndrome, trisomy 22, trisomy 16, 1q21.1 deletion syndrome, 1q21.1 duplication syndrome, TAR syndrome, 1p36 deletion syndrome, Wolf-Hirschhorn syndrome, cri du chat syndrome, chromosome 5q deletion syndrome, Williams syndrome, Jacobsen syndrome, Miller-Dieker syndrome, Smith-Magenis syndrome, DiGeorge syndrome, 22q11.2 distal deletion syndrome, 22q13 deletion syndrome, Angelman syndrome, Prader-Willi syndrome, distal 18q-, proximal 18q-, Turner syndrome, Klinefelter syndrome, XXYY syndrome, XXXY syndrome, 49XXXYY syndrome, 49XXXXY syndrome, triple X syndrome, tetrasomy X, 49XXXXX, Jacobs syndrome, 48XYYY, 49XYYYY, 45X/46XY, 46XX/46XY, and the like. Other genetic events may include spina bifida, cleft palate, Tay-Sachs disease, sickle cell anemia, thalassemia, cystic fibrosis, muscular dystrophy, fragile X syndrome.

According to the invention, the method comprises a step of pooling individual samples from each subject of the population of subjects, to obtain a pooled sample, hereinafter also called "sample". Nevertheless, in a preferred embodiment, the method according to the invention is performed on a sample from a single subject.

In one embodiment, the subjects are animals. Examples of animals include, but are not limited to, mammals, birds, reptiles, amphibians, fishes, insects and mollusks.

In one embodiment, the subjects are humans.

In one embodiment, the subjects are at risk of suffering from a genetic event.

In one embodiment, a population of subjects comprises at least 2 subjects or individuals.

In one embodiment, a population of subjects comprises from 2 to 1500 subjects or individuals, preferably from 2 to 1000, from 2 to 500, from 2 to 400, from 2 to 300, from 2 to 200, from 2 to 100 subjects or individuals.

In one embodiment, a population of subjects comprises from 2 to 96 subjects or individuals, preferably from 2 to 88, from 2 to 80, from 2 to 72, from 2 to 64, from 2 to 56, from 2 to 48, from 2 to 40, from 2 to 32, from 2 to 24, from 2 to 16, from 2 to 8 subjects or individuals.

In one embodiment, a population of subjects comprises from 4 to 96 subjects or individuals, preferably from 4 to 88, from 4 to 80, from 4 to 72, from 4 to 64, from 4 to 56, from 4 to 48, from 4 to 40, from 4 to 32, from 4 to 24, from 4 to 16, from 4 to 8 subjects or individuals.

In one embodiment, a population of subjects comprises from 6 to 96 subjects or individuals, preferably from 6 to 88, from 6 to 80, from 6 to 72, from 6 to 64, from 6 to 56, from 6 to 48, from 6 to 40, from 6 to 32, from 6 to 24, from 6 to 16, from 6 to 8 subjects or individuals.

In one embodiment, a population of subjects comprises from 8 to 96 subjects or individuals, preferably from 8 to 88, from 8 to 80, from 8 to 72, from 8 to 64, from 8 to 56, from 8 to 48, from 8 to 40, from 8 to 32, from 8 to 24, from 8 to 16 subjects or individuals.

Examples of suitable samples include, but are not limited to, blood, plasma, serum, lymph, ascetic fluid, cystic fluid, urine, bile, nipple exudate, synovial fluid, nasopharyngeal swabs, nasopharyngeal wash, oropharyngeal swabs, oropharyngeal wash, sputum, endotracheal aspirates, bronchoalveolar lavage fluids, amniotic fluid, peritoneal fluid, cerebrospinal fluid, pleural fluid, pericardial fluid, semen, saliva, sweat and alveolar macrophages.

In one embodiment, the sample is selected from the group comprising or consisting of nasopharyngeal swabs, nasopharyngeal aspirates, nasopharyngeal wash, oropharyngeal swabs, oropharyngeal aspirates, oropharyngeal wash, sputum, endotracheal aspirates, bronchoalveolar lavage fluids, and mixes thereof.

In one embodiment, the sample is a nasopharyngeal swab and/or a nasopharyngeal aspirate.

In one embodiment, the sample is selected from the group comprising or consisting of blood, plasma, serum, amniotic fluid, cerebrospinal fluid, urine, feces, saliva and mixes thereof.

In one embodiment, the method described herein does not include a step of collecting the sample in the subject, i.e., the method is an *in vitro* method which can be performed in absence of the subject.

The methods described herein may comprise one or more of sample preparation steps, including, but not limited to, extracting nucleic acid molecules from the sample, fragmenting nucleic acid molecules, concentrating nucleic acid molecules and/or diluting nucleic acid molecules. These optional sample preparation steps can be carried on each individual sample, and/or on the pooled sample. One skilled in the art is familiar with these sample preparation steps.

In one embodiment, these optional sample preparation steps are carried out on the pooled sample. In particular, in one embodiment, nucleic acid molecules are extracted from the pooled sample.

In one embodiment, only same or similar types of individual samples are pooled together.

In one embodiment, the same amount, volume or weight of individual samples are pooled together.

In one embodiment, the sample is diluted to a final volume ranging from about 500 $\mu$L to about 10 mL, from about 500 $\mu$L to about 5 mL, from about 500 $\mu$L to about 2 mL, from about 1 mL to about 10 mL, from about 1 mL to about 5 mL, from about 1 mL to about 2 mL.

According to the invention, the method comprises a step of extracting nucleic acids.

Extracting nucleic acids can be carried out by methods well known to the one skilled in the art. Such methods include, but are not limited to, chloroform-isoamyl alcohol extraction, phenol-chloroform extraction, alkaline extraction, guanidinium thiocyanate-phenol-chloroform extraction, binding on anion exchange resin, silica matrices, glass particle, diatomaceous earth, magnetic particles made from different synthetic polymers, biopolymers, porous glass and based on inorganic magnetic.

Advantageously, nucleic acids are extracted from the sample. In particular, it is advantageous to extract nucleic acids from a large volume of sample, e.g., from about 500 $\mu$L to about 10 mL, from about 500 $\mu$L to about 5 mL, from about 500 $\mu$L to about 2 mL, from about 1 mL to about 10 mL, from about 1 mL to about 5 mL, from about 1 mL to about 2 mL, which minimizes the loss of material during sample handling.

According to the invention, the method comprises a step of partitioning the sample and/or extracted nucleic acids into a plurality of partitions, wherein said partitions comprise an amplification mixture comprising a polymerase; at least two pairs of nucleic acid primers capable of hybridizing to at least two nucleic acid targets of a genetic event; and at least two nucleic acid probes attached or linked to a detectable label.

According to the invention, each of the at least two pairs of nucleic acid primers is capable of hybridizing to a respective nucleic acid target of a same genetic event.

According to the invention, each of the at least two nucleic acid probes is capable of hybridizing to a different nucleic acid target of the same genetic event and/or to its amplification product.

According to the invention, the at least two nucleic acid targets are located, upon spontaneous and/or artificial fragmentation of the nucleic acids in the sample, on separate fragments of said nucleic acids.

The partitions of the plurality of partitions can be in any suitable format. Microwell plates, capillaries, oil emulsion, and arrays of miniaturized chambers with nucleic acid binding surfaces can be used to partition the samples in distinct partitions or droplets. Thus, digital PCR, as used herein, includes a variety of formats, including crystal digital PCR™, chamber digital PCR, droplet digital™ PCR (ddPCR), BEAMing (beads, emulsion, amplification, and magnetic), and microfluidic chips.

The samples and/or extracted nucleic acids can be partitioned into a plurality of partitions, which can be advantageous to reduce background amplification, reduce amplification bias, increase throughput, provide absolute or relative quantitative detection, or a combination thereof. Partitions can include any of a number of types of partitions, including solid partitions (e.g., wells or tubes) or fluid partitions (e.g., aqueous droplets within an oil phase).

In one embodiment, the plurality of partitions comprises or consists of a plurality of droplets. In one embodiment, the plurality of partitions is arranged in a 1-dimensional array of droplets in a microchannel. In one embodiment, the plurality of partitions is arranged in a 2-dimensional array of droplets (i.e., a monolayer) in a microchamber.

In one embodiment, the sample and/or extracted nucleic acids is/are partitioned into a plurality of droplets. In one embodiment, a droplet comprises an emulsion composition, i.e., a mixture of immiscible fluids (e.g., water and oil). In one embodiment, a droplet is an aqueous droplet that is surrounded by an immiscible carrier fluid (e.g., oil). In one embodiment, a droplet is an oil droplet that is surrounded by an immiscible carrier fluid (e.g., an aqueous solution).

In one embodiment, the droplets described herein are relatively stable and have minimal coalescence between two or more droplets. In one embodiment, less than 0.0001 %, 0.0005 %, 0.001 %, 0.005 %, 0.01 %, 0.05 %, 0.1 %, 0.5 %, 1 %, 2 %, 3 %, 4 %, 5 %, 6 %, 7 %, 8 %, 9 %, or 10 % of droplets generated from the sample and/or extracted nucleic acids coalesce with other droplets. The emulsions can also have limited flocculation, a process by which the dispersed phase comes out of suspension in flakes.

In one embodiment, the droplets are formed by flowing an oil phase against the sample and/or extracted nucleic acids. In one embodiment, the droplets are formed by flowing the sample and/or extracted nucleic acids through microchannels comprising wall portions that diverge to detach a droplet of the sample and/or extracted nucleic acids under the effect of surface tension of the solution into a storage zone with an oil phase carrier fluid in a microfluidic device. The oil phase can comprise a fluorinated base oil, which can additionally be stabilized by combination with a fluorinated surfactant such as a perfluorinated polyether. In one embodiment, the oil phase further comprises an additive for tuning the oil properties, such as vapor pressure, viscosity, or surface tension.

In one embodiment, the emulsion is formulated to produce highly monodisperse droplets having a liquid-like interfacial film that can be converted by heating into microcapsules having a solid like interfacial film; such microcapsules can behave as bioreactors able to retain their contents through an incubation period. The conversion to microcapsule form can occur upon heating. For example, such conversion can occur at a temperature of greater than about 40°C, 50°C, 60°C, 70°C, 80°C, 90°C, or 95°C. During the heating process, a fluid or mineral oil overlay can be used to prevent evaporation. Excess continuous phase oil can be removed prior to heating, or not. The microcapsules can be resistant to coalescence and/or flocculation across a wide range of thermal and mechanical processing. In some embodiments, these capsules are useful for storage or transport of partition mixtures.

In one embodiment, the sample is partitioned into at least 500 partitions, at least 1000 partitions, at least 2000 partitions, at least 3000 partitions, at least 4000 partitions, at least 5000 partitions, at least 6000 partitions, at least 7000 partitions, at least 8000 partitions, at least 10000 partitions, at least 15000 partitions, at least 20000 partitions, at least 30000 partitions, at least 40000 partitions, at least 50000 partitions, at least 60000 partitions, at least 70000 partitions, at least 80000 partitions, at least 90000 partitions, at least 100,000 partitions, at least 200,000 partitions, at least 300000 partitions, at least 400000 partitions, at least 500000 partitions, at least 600000 partitions, at least 700000 partitions, at least 800000 partitions, at least 900000 partitions, at least 1000000 partitions, at least 2000000 partitions, at least 3000000 partitions, at least 4000000 partitions, at least 5000000 partitions, at least 10000000 partitions, at least 20000000 partitions, at least 30000000 partitions, at least 40000000 partitions, at least 50000000 partitions, at least 60000000 partitions, at least 70000000 partitions, at least 80000000 partitions, at least 90000000 partitions, at least 100000000 partitions, at least 150000000 partitions, or at least 200000000 partitions.

In one embodiment, the naica® System (Stilla Technologies) is used to carry out the methods described herein. In one embodiment, the Sapphire Chip of the naica® System (Stilla Technologies) is used to partition the sample and/or extracted nucleic acids. Typically, a Sapphire Chip contains 4 microchambers, each with a droplet crystal, i.e., a self-assembled array of droplets.

In one embodiment, the sample and/or extracted nucleic acids is/are partitioned into a sufficient number of partitions such that at least a majority of partitions has no more than 0 to 5 nucleic acid targets, preferably no more than 1 to 5 nucleic acid targets (e.g., no more than about 1, 2, 3, 4, or 5 nucleic acid targets). By "a **majority of partitions",** it is meant at least 50 %, at least 55 %, at least 60 %, at least 65 %, at least 70 %, at least 75 %, at least 80 %, at least 85 %, at least 90 %, at least 95 %, or more, of the partitions in the population of partitions. Preferably, the sample and/or extracted nucleic acids is/are partitioned into a sufficient number of partitions such that at least a majority of partitions has no more than 0 to 2 nucleic acid targets. Preferably, the sample and/or extracted nucleic acids is/are partitioned into a sufficient number of partitions such that at least a majority of partitions has no more than 1 to 2 nucleic acid targets.

In one embodiment, the sample and/or extracted nucleic acids is/are not partitioned into too many partitions, such that a minority of partitions has no nucleic acid targets. By **"a minority of partitions"**, it is meant at most 50 %, at most 45 %, at most 40 %, at most 35 %, at most 30 %, at most 25 %, at most 20 %, at most 15 %, at most 10 %, at most 5 %, or less, of the partitions in the population of partitions.

In one embodiment, the droplets that are generated are substantially uniform in shape, size and/or volume. For example, in one embodiment, the droplets are substantially uniform in average diameter. In one embodiment, the droplets that are generated have an average diameter of about 0.001 μm, about 0.005 μm, about 0.01 μm, about 0.05 μm, about 0.1 μm, about 0.5 μm, about 1 μm, about 5 μm, about 10 μm, about 20 μm, about 30 μm, about 40 μm, about 50 μm, about 60 μm, about 70 μm, about 80 μm, about 90 μm, about 100 μm, about 150 μm, about 200 μm, about 300 μm, about 400 μm, about 500 μm, about 600 μm, about 700 μm, about 800 μm, about 900 μm, or about 1000 μm. In one embodiment, the droplets that are generated have an average diameter of less than about 1000 μm, less than about 900 μm, less than about 800 μm, less than about 700 μm, less than about 600 μm, less than about 500 μm, less than about 400 μm, less than about 300 μm, less than about 200 μm, less than about 100 μm, less than about 50 μm, or less than about 25 μm. In one embodiment, the droplets that are generated are non-uniform in shape and/or size.

In one embodiment, the droplets that are generated are substantially uniform in volume. For example, the standard deviation of droplet volume can be less than about 1 pL, 5 pL, 10 pL, 100 pL, 1 nL, or less than about 10 nL. In some cases, the standard deviation of droplet volume can be less than about 10-25% of the average droplet volume. In one embodiment, the droplets that are generated have an average volume of about 0.001 nL, about 0.005 nL, about 0.01 nL, about 0.02 nL, about 0.03 nL, about 0.04 nL, about 0.05 nL, about 0.06 nL, about 0.07 nL, about 0.08 nL, about 0.09 nL, about 0.1 nL, about 0.2 nL, about 0.3 nL, about 0.4 nL, about 0.5 nL, about 0.6 nL, about 0.7 nL, about 0.8 nL, about 0.9 nL, about 1 nL, about 1.5 nL, about 2 nL, about 2.5 nL, about 3 nL, about 3.5 nL, about 4 nL, about 4.5 nL, about 5 nL, about 5.5 nL, about 6 nL, about 6.5 nL, about 7 nL, about 7.5 nL, about 8 nL, about 8.5 nL, about 9 nL, about 9.5 nL, about 10 nL, about 11 nL, about 12 nL, about 13 nL, about 14 nL, about 15 nL, about 16 nL, about 17 nL, about 18 nL, about 19 nL, about 20 nL, about 25 nL, about 30 nL, about 35 nL, about 40 nL, about 45 nL, or about 50 nL.

According to the invention, the partitions comprise an amplification mixture, comprising a polymerase; at least two pairs of nucleic acid primers, each capable of hybridizing to a respective nucleic acid target of a genetic event; and at least two nucleic acid probes attached or linked to a detectable label.

According to the invention, each of the at least two pairs of nucleic acid primers is capable of hybridizing to a different nucleic acid target of the same genetic event.

According to the invention, each of the at least two nucleic acid probes is capable of hybridizing to a different nucleic acid target of the same genetic event and/or to its amplification product.

According to the invention, the at least two nucleic acid targets are located, upon spontaneous and/or artificial fragmentation of the nucleic acids in the sample, on separate fragments of said nucleic acids.

The amplification mixture can be incorporated into the sample and/or extracted nucleic acids, and the sample and/or extracted nucleic acids is/are then partitioned into a plurality of partitions. Alternatively, the sample and/or extracted nucleic acids can be partitioned into a plurality of partitions and the amplification mixture is incorporated into the partitioned samples and/or extracted nucleic acids.

According to the invention, the amplification mixture comprises a polymerase, which is an enzyme that performs template-directed synthesis of nucleic acids, e.g., DNA and/or RNA. The term **"polymerase"** encompasses both the full-length enzyme and a domain that has a polymerase activity. Polymerases are well known to those skilled in the art, including but not limited to, DNA polymerases isolated or derived from *Pyrococcus furiosus*, *Thermococcus litoralis,* and *Thermotoga maritime,* or modified versions thereof. Additional examples of commercially available polymerase enzymes include, but are not limited to, Klenow fragment, Taq DNA polymerase, 9°WM DNA polymerase, Deep Vent™ DNA polymerase, Manta DNA polymerase, Bst DNA polymerase, phi29 DNA polymerase, Takyon™ DNA polymerase (Eurogentec; EP2240576), AccuStart II Taq DNA (Quanta Bio).

In one embodiment, the polymerase is a DNA-dependent polymerase. In one embodiment, the polymerase is an RNA-dependent polymerase, such as a reverse transcriptase.

In one embodiment, the amplification mixture may comprise both a DNA-dependent polymerase and an RNA-dependent polymerase. In particular, such mix is particularly suitable to reverse-transcribe an RNA target to cDNA, then amplify the reverse-transcribed cDNA target.

According to the invention, the amplification mixture comprises at least two pairs of nucleic acid primers, each capable of hybridizing to a respective nucleic acid target of a genetic event.

According to the invention, each of the at least two pairs of nucleic acid primers is capable of hybridizing to a different nucleic acid target of the same genetic event.

A pair of nucleic acid primers typically comprises a forward primer and a reverse primer, that anneal to opposite strands of a nucleic acid molecule and flank the nucleic acid target. Each pair of nucleic acid primers thus allows the production of an amplicon (or amplification product) specific to the respective nucleic acid target to which it hybridizes, if present, during an amplification assay, such as, e.g., a digital PCR assay.

In one embodiment, the amplification mixture comprises multiple clonal copies of each pair of nucleic acid primers. By **"clonal copies",** it is meant a population of several hundreds, thousands, tens of thousands, hundreds of thousands, millions or more identical copies of nucleic acid primers, i.e., having the same nucleic acid sequence, or substantially the same nucleic acid sequence as long as they hybridize to the same nucleic acid targets.

The one skilled in the art is familiar with nucleic acid primer design for amplification purposes. The nucleic acid primers may be of any suitable length and GC contents. In one embodiment, a pair of nucleic acid primers can be designed using available computer programs, such that upon amplification, the resulting amplicons are predicted to have the same melting temperature.

In one embodiment, the nucleic acid primers are designed so as to provide an amplicon having a suitable length, so that, e.g., the amplicon is long enough to allow hybridization with a nucleic acid probe. In one embodiment, the amplicons have a length of at least about 50, 100, 150, 200, 250, 300, 350, 400, 450, or 500 basepairs. In one embodiment, the amplicons have a length of no more than about 500, 450, 400, 350, 300, 250, 200, 150, or 100 basepairs. In one embodiment, the amplicons have a length ranging from about 100 to about 500, from about 100 to about 400, from about 100 to about 300, from about 100 to about 250, from about 100 to about 200, from about 150 to about 250, or from about 150 to about 300 basepairs long.

In one embodiment, each partition comprises two or more pairs of nucleic acid primers, in particular 2, 3, 4, 5, or 6, each pair being capable of hybridizing to a different nucleic acid target of a genetic event.

In one embodiment, each partition comprises three or more pairs of nucleic acid primers, in particular 3, 4, 5, or 6, each pair being capable of hybridizing to a different nucleic acid target of a genetic event.

In one embodiment, the two or more pairs of nucleic acid primers are designed and/or selected in such a way that their corresponding nucleic acid targets are located, upon spontaneous and/or artificial fragmentation of nucleic acids representative of a genetic event, on separate fragments of said nucleic acids.

Indeed, in digital PCR assays, a majority of partitions in the plurality of partitions typically contains either 0 or 1 nucleic acid molecules, although the person skilled in the art is aware that a proportion of partitions, typically less than 50 % of the total partitions, may contain more than 1 nucleic acid fragment, such as 2, 3, 4 nucleic acid fragments or even more in rare instances. These nucleic acid molecules are, typically, not full-length genomes, but fragmented pieces of nucleic acids. This fragmentation can occur spontaneously upon sample processing (during pipetting, vortexing, heating and the like) and/or can be induced by contacting the sample with endonucleases, such as, *e.g.*, sequence-specific endonucleases.

Preferably, such fragmentation occurs spontaneously within a subject. This increases the probability that a nucleic acid target, if present in the subject, will be detected in at least one partition. Advantageously, fragmentation occurs spontaneously in a large volume, *e.g.*, between 1 mL and 10 L.

It is desirable, in order to improve the sensitivity and the precision of the method described herein, that multiple nucleic acid targets be targeted, although these targets then need to be located on separate fragments of the fragmented nucleic acids representative of the genetic event, so as to behave independently from each other during partitioning of the sample into a plurality of partitions, and therefore behave independently from each other in the digital PCR assay. In other words, when nucleic acid fragments statistically behave independently from each other, doubling the number of nucleic acid targets targeted in the assay amounts to doubling the sensitivity of the assay in the analyzed sample by virtually doubling the concentration of nucleic acids representative of a genetic event in the sample, i.e., by dividing by 2 the limit of detection (LOD) of the assay in the analyzed sample. This is in clear contrast with qPCR techniques currently carried out, where multiplying the number of nucleic acid targets does not increase the sensitivity of the test.

By **"statistically behave independently from each other",** it is meant that the probability of finding a given nucleic acid target in any particular partition is independent of the presence or absence of another nucleic acid target in any partition; or in other words, the occupancies of the partitions by nucleic acid targets are independent of each other.

Based on the above, the present invention also encompasses means and methods for improving the sensitivity and the precision of a digital PCR assay, regardless of the purpose of the test, *i.e.,* the rationale underlying the increased sensitivity and increased precision of the digital PCR test can be exploited in any test which requires a high sensitivity to detect a genetic event and/or a high precision to quantify a nucleic acid target. In particular, the one skilled in the art will readily understand that the means and methods for improving the sensitivity and the precision of a digital PCR assay disclosed herein can also be exploited, *e.g.*, for the detection of a rare genetic event. The means and methods for improving the sensitivity and the precision of a digital PCR assay disclosed herein can also be exploited if the nucleic acid targets of the genetic event are present at a very low concentration in the sample, which is often the case for the nucleic acids of an embryo or a fetus in a sample from the pregnant individual carrying said embryo or fetus.

Based on these observations, the Applicant has carried out tests further described in Example 2 below, using two different targets on the SARS-CoV-2 genome, sufficiently distant from each other in the genome, so as to be able to confirm by statistical analysis that, in droplets encapsulating the two distinct nucleic acid targets, the two fragments of nucleic acid behave independently from each other, *i.e.,* they are not co-encapsulated in a single partition more frequently than expected for two distinct molecules. This allows to confirm that the two nucleic acid targets of Example 2 are indeed located on two sections of the SARS-CoV-2 genome which are always fragmented into independent fragments during experimental processing. That is, the occurrence of partitions in which the two nucleic acid targets are detected are not statistically over-represented (due to, *e.g.*, cleavage issues).

Additionally, it is desirable that pairs of nucleic acid primers be designed and/or selected in such a way that their nucleic acid targets be located in genomic regions which are not prone to spontaneous mutations.

One skilled in the art can therefore readily design and select other and/or further pairs of nucleic acid primers targeting other and/or further nucleic acid targets, based on these considerations, and further validate by statistical analysis that each nucleic acid target behaves independently from each other.

In particular, one skilled in the art can take into account the distance between the 3' end of a first nucleic acid target and the 5' end of a second nucleic acid target, so as to ensure that each target statistically behaves independently from each other. In one embodiment, this distance is at least 2000 bases, at least 2500 bases, at least 3000 bases, at least 3500 bases, at least 4000 bases, at least 4500 bases, at least 5000 bases, at least 5500 bases, at least 6000 bases, at least 6500 bases, at least 7000 bases, at least 7500 bases, at least 8000 bases, at least 8500 bases, at least 9000 bases, at least 9500 bases, at least 10000 bases.

Additionally, or alternatively, one skilled in the art can take into account the three-dimensional folding of the genome (in particular in the case of an RNA genome), and select targets located in different structural regions/domains and/or separated by unstructured regions/domains which are prone to spontaneous fragmentation, so as to ensure that each target statistically behaves independently from each other.

Additionally, or alternatively, one skilled in the art can run assays on the nucleic acids representative of a genetic event to determine their spontaneous fragmentation pattern, such as, *e.g.*, Southern blots (for a DNA genome) or Northern blots (for an RNA genome); and with this information in hand, design or select pairs of nucleic acid primers targeting nucleic acid targets located on separate fragments of the fragmented nucleic acids.

Additionally, or alternatively, one skilled in the art can design or select pairs of nucleic acid primers targeting nucleic acid targets located on both sides of an identified endonuclease cleaving site in the nucleic acids representative of a genetic event, so as to ensure that each target statistically behaves independently from each other.

An **"endonuclease cleaving site"** can be a nucleic acid sequence pattern or motif, or a specific nucleic acid structure.

In one embodiment, the optional step of fragmenting the nucleic acids representative of the genetic event can be carried out before the step of partitioning the sample into a plurality of partitions.

Advantageously, spontaneous and/or artificial fragmentation of the nucleic acids occurs in the sample, before the step of partitioning the extracted nucleic acids into a plurality of partitions, that is to say, in a sample volume that is significantly larger than the final volume analyzed by digital PCR. In this case, detecting the presence of a genetic event with k nucleic acid targets that are equally amplifiable and 100% amplifiable permits to divide the Limit Of Detection (LOD) of the nucleic acids representative of the genetic event by a factor $k$ *(i.e.,* sensitivity of the digital PCR test is increased $k$ times), as is explained in more details below.

In one embodiment, the method comprises a step of artificially fragmentating nucleic acids in the sample, in conditions suitable to have the at least two nucleic acid targets being located on separate fragments of said nucleic acids, concomitantly with or after the step of extracting nucleic acids from the sample but before the step of partitioning the extracted nucleic acids into a plurality of partitions. In this embodiment, the at least two nucleic acid targets behave statistically independently from each other during partitioning of the sample into a plurality of partitions, and therefore behave independently from each other during the step of performing a digital PCR assay.

In one embodiment, the method according to the invention comprises a step of artificially fragmentating nucleic acids in the sample by contacting the sample with at least one sequence-specific endonuclease.

In one embodiment, the at least one sequence-specific endonuclease is selected from sequence-specific endonucleases capable of cleaving the nucleic acids between the at least two nucleic acid targets, thereby having the at least two nucleic acid targets located on separate fragments of said nucleic acids upon artificial fragmentation and statistically behaving independently from each other during partitioning of the sample into a plurality of partitions, and therefore behaving independently from each other during the dPCR assay.

By **"sequence-specific endonuclease",** it is meant an endonuclease with a specificity for an identified endonuclease cleaving site in the nucleic acids.

Examples of sequence-specific endonucleases are well known to the one skilled in the art. They include in particular restriction enzymes, i.e., enzymes that cleave DNA and/or RNA into fragments at or near specific recognition sites within molecules known as **"restriction sites".** The REBASE® or "Restriction Enzyme Database", accessible at http://rebase.neb.com/rebase/rebase.html, provides thousands of restriction enzymes and their restriction sites.

In one embodiment, the step of fragmenting the nucleic acids representative of the genetic event can be carried out before or after the step of pooling individual samples from each subject of the population of subjects, to obtain a pooled sample.

In one embodiment, the step of fragmenting the nucleic acids representative of the genetic event can be carried out before or after the step of partitioning the pooled sample into a plurality of partitions.

In the latter, the amplification mixture further comprises at least one endonuclease, preferably at least one endonuclease with a specificity for an identified endonuclease cleaving site in the nucleic acids representative of the genetic event.

One skilled in the art will readily recognize that endonuclease shall preferably be inactivated before initiation or completion of the step of performing a digital polymerase chain reaction (dPCR) on the plurality of partitions, so as to avoid any non-specific cleaving of nucleic acids in the sample. In one embodiment, endonucleases can be heat-inactivated. In one embodiment, endonucleases are heat-inactivated during the step of performing a digital polymerase chain reaction (dPCR) on the plurality of partitions.

According to the invention, the amplification mixture comprises at least two, in particular 2, 3, 4, 5, 6 or more, nucleic acid probes attached or linked to a detectable label.

According to the invention, each nucleic acid probe is capable of hybridizing to a different nucleic acid target of the same genetic event, and/or to its amplification product.

In one embodiment, the nucleic acid probe has a nucleic acid sequence that is specific for a nucleic acid target, and is capable of annealing with an amplicon (or amplification product) amplified by a specific pair of nucleic acid primers during an amplification assay, such as, e.g., a digital PCR assay.

Different types of nucleic acid probes are available and well known to the skilled artisan.

Exemplary nucleic acid probes may comprise an oligonucleotide attached to a detectable, fluorescent label, and further to a fluorescence quencher, wherein the detectable, fluorescent label and the quencher are in proximity until the nucleic acid probe specifically binds to an amplicon (or amplification product) (*e.g.*, TaqMan™ probes, "Scorpions® probes", "molecular beacons", and the like). Such nucleic acid probes may be used to measure the total amount of reaction product at the completion of an assay, or to measure the generation of amplicons (or amplification product) during an amplification reaction, such as, e.g., a digital PCR assay.

In one embodiment, the nucleic acid probes are TaqMan™ probes.

The quencher may be an internal quencher or a quencher located at the 3' end of the nucleic acid probe. Typical quenchers include, but are not limited to, tetramethylrhodamine, TAMRA, BLACK HOLE QUENCHER® (BHQ, e.g., BHQ-1, BHQ-2, BHQ-3), and nonfluorescent quencher (NFQ). Hydrolysis probes usable according to the invention are well known in the field. In one embodiment, hydrolysis probes have a fluorescent label covalently attached to the 5'end of the nucleic acid probe and a quencher. The quencher molecule quenches the fluorescence emitted by the fluorescent label when excited by a light source, typically via FRET (Forster Resonance Energy Transfer). As long as the fluorescent label and the quencher are in proximity, quenching inhibits any fluorescence signals. In one embodiment, the nucleic acid probes are designed such that they anneal within the nucleic acid target amplified by a specific pair of nucleic acid primers. As the DNA polymerase extends the primer and synthesizes the nascent strand, the 5'-3' exonuclease activity inherent in the DNA polymerase then separates the 5' fluorescent label from the 3' quencher, which provides a fluorescence signal that is proportional to the amplicon yield.

In one embodiment, the nucleic acid probes have a nucleotide sequence length of about 10 to about 50 bases. In some embodiments, the nucleic acid probes have a nucleotide sequence length of about any one of 15-40, 25-50, 15-35, 20-40, 30-50 or 30-40 bases.

In one embodiment, the amplification mixture comprises multiple clonal copies of each nucleic acid probe, that is to say, a population of several hundreds, thousands, tens of thousands, hundreds of thousands, millions or more identical copies of each nucleic acid probe, *i.e.*, having the same nucleic acid sequence, or substantially the same nucleic acid sequence as long as they hybridize to the same nucleic acid targets and/or its amplification product. Clonal copies of a given nucleic acid probe may however be attached or linked to the same or different detectable labels.

The nucleic acid probes may further comprise modifications that increase the specificity of the nucleic acid probes to their nucleic acid target, *e.g.,* by increasing the melting temperature (Tm) of the nucleic acid probe and stabilizing nucleic acid probe- nucleic acid target hybrids.

In one embodiment, a nucleic acid probe may include a minor groove binder (MGB) moiety at its 3' end. In one embodiment, a nucleic acid probe may comprise a chemically modified nucleotide, such as a Locked Nucleic Acid (LNA).

In one embodiment, the nucleic acid probes are attached or linked to a detectable label.

Examples of suitable detectable labels include, but are not limited to, FAM (5- or 6- carboxyfluorescein), HEX, CY5, VIC, NED, fluorescein, FITC, IRD-700/800, CY3, CY3.5, CY5.5, TET (5-tetrachloro-fluorescein), TAMRA, JOE, ROX, BODIPY TMR, Oregon Green, Rhodamine Green, Rhodamine Red, Texas Red, Yakima Yellow, Alexa Fluor PET, BIOSEARCH BLUE™, MARINA BLUE®, BOTHELL BLUE®, ALEXA FLUOR®, 350 FAM™, SYBR® Green 1, EvaGreen™, ALEXA FLUOR® 488 JOE™, 25 VIC™, HEX™, TET™, CAL FLUOR® Gold 540, YAKIMA YELLOW®, ROX™, CAL FLUOR® Red 610, Cy3.5™, TEXAS RED®, ALEXA FLUOR® 568 CRY5™, QUASAR™ 670, LIGHTCYCLER RED640®, ALEXA FLUOR® 633 QUASAR™ 705, LIGHTCYCLER RED705®, ALEXA FLUOR® 680, SYT0®9, LC GREEN®, LC GREEN® Plus+, and EVAGREEN™.

In one embodiment, the detectable label is selected from the group comprising or consisting of fluorescein, FAM, Yakim Yellow, Cy3, HEX, VIC, ROX, CY5, CY5.5, Alexa Fluor 647, AlexaFluor448, and Quasar705.

In one embodiment, the detectable label is selected from the group comprising or consisting of Cy3, FAM and Cy5. In one embodiment, the detectable label is selected from the group comprising or consisting of FAM, HEX and Cy5.

In one embodiment, at least two nucleic acid probes, each capable of hybridizing to a different nucleic acid target and/or to its amplification product, are attached or linked to detectable labels having non-overlapping excitation wavelength ranges and/or non-overlapping emission wavelength ranges with respect to the other detectable label(s).

In this embodiment, each of the at least two nucleic acid probes is attached or linked to a different detectable label.

In this embodiment, each of the at least two nucleic acid probes may be detected, upon binding to its nucleic acid target and/or to its amplification product, via different detection channels, each having a characteristic excitation range and/or a characteristic emission range.

In one embodiment, the nucleic acid probe has a nucleic acid sequence that is specific for a nucleic acid target, and is capable of annealing with an amplicon (or amplification product) amplified by a specific pair of nucleic acid primers during an amplification assay, such as, *e.g.*, a digital PCR assay.

In one embodiment, each nucleic acid probe being specific for a given nucleic acid target, if more than one, may or not be detected via a detection channel having a characteristic excitation range and a characteristic emission range. In one embodiment, each nucleic acid probe being specific for a given nucleic acid target, if more than one, may or not have non-overlapping excitation wavelength ranges and/or non-overlapping emission wavelength ranges with the other nucleic acid probes.

In one embodiment, the amplification mixture may comprise, apart from the polymerase, nucleic acid primers and nucleic acid probes, any other reagents including, but not limited to, enzymes; substrates (such as, *e.g.*, a mix of 4 deoxyribonucleotides triphosphate (dNTPs), namely deoxyadenosine triphosphate (dATP), deoxycytidine triphosphate (dCTP), deoxyguanosine triphosphate (dGTP) and (deoxy)thymidine triphosphate (dTTP)); buffers; salts (such as, e.g., divalent cations including $Mg^{2+}$); additives (such as, *e.g.*, betaine, BSA (A-420), DMSO, DTT, formamide, glycerol, polyethylene glycol, 1,2-propanediol, tetramethylammonium chloride, and the like); detergents; and the like.

According to the invention, the method comprises a step of performing a digital polymerase chain reaction (dPCR) on the plurality of partitions under conditions suitable to amplify, if present, at least two nucleic acid targets of the genetic event.

When in the "digital" range, it is possible to multiplex amplification assays without concern for competition or cross reactivity, as each nucleic acid target-containing partition will proceed with the nucleic acid target binding to its pair of nucleic acid primers and optionally, of nucleic acid probe specifically; whereas no reaction will occur in partitions without nucleic acid target. This is because a majority of partitions typically contains either 0 or 1 nucleic acid fragment (*i.e.*, when the nucleic acids are spontaneously or artificially fragmented as defined above, a majority of partitions typically contains either 0 or 1 nucleic acid target), although the skilled artisan is aware that a proportion of partitions, typically less than 50 % of the total partitions, may contain more than 1 nucleic acid fragment, such as 2, 3, 4 nucleic acid fragments or even more in rare instances. Having a single nucleic acid fragment per partition in a large number of partitions allows both high and low abundance nucleic acid targets to be counted in the same experiment without concern for "swamping out" the low abundance target.

Techniques available for digital PCR include PCR amplification on a microfluidic chip. Other systems involve separation onto microarrays or spinning microfluidic discs, and droplet techniques based on oil-water emulsions. Typically, digital PCR is selected from crystal digital PCR™, droplet digital™ PCR (ddPCR), BEAMing (beads, emulsion, amplification, and magnetic), and microfluidic chips.

In one embodiment, the dPCR is crystal digital PCR™.

Examples of suitable droplet digital PCR systems include the naica® system for Crystal Digital PCR™ by Stilla Technologies, which partitions samples into 25000-30000 nanoliter-sized droplets; QX100™ Droplet Digital™ PCR System by Bio-Rad, which partitions samples containing nucleic acid template into 20000 nanoliter-sized droplets; and the RAINDROP™ digital PCR system by RainDance, which partitions samples containing nucleic acid template into 1000000 to 10000000 picoliter-sized droplets.

According to the invention, the method comprises a step of detecting a fluorescence signal in the plurality of partitions, wherein the fluorescence signal in a given partition is indicative of amplification of a nucleic acid target of the genetic event in said given partition.

As detailed hereinabove, it is desirable, in order to improve the sensitivity and the precision of the method described herein, that multiple nucleic acid targets be targeted. As will be further detailed below and as already mentioned above, the use of multiple nucleic acid targets - located, upon spontaneous and/or artificial fragmentation of the nucleic acids, on separate fragments of said nucleic acids so as to behave statistically independently from each other during partitioning of the sample into a plurality of partitions, and therefore behave independently from each other during the dPCR assay - allows to improve the sensitivity and the precision of the method described herein but also of any methods of analyzing a sample by digital PCR, regardless of the purpose of the test, *i.e.,* the rationale underlying the increased sensitivity and precision of the digital PCR test can be exploited in any test which requires a high sensitivity for detecting a genetic event and/or a high precision for quantifying a nucleic acid target.

In a first embodiment where at least two nucleic acid targets are targeted (each being capable of hybridizing to a different nucleic acid target of the same genetic event and/or to its amplification product), both or all of the at least two nucleic acid probes may be detected via the same detection channel *(i.e.,* at the same excitation range and emission range, or in other words, with overlapping excitation and emission wavelength ranges).

In this first embodiment, one detection channel *(i.e.,* with a given excitation/emission wavelength) can detect several nucleic acid targets. In the event that these nucleic acid targets behave independently from each other - as explained hereinabove -, and by summing the number of positive events detected in one channel, the sensitivity of the assay can be multiplied by up to the number of independent nucleic acid targets, *i.e.,* the limit of detection (LOD) of the assay can be divided by up to the number of independent nucleic acid targets.

In this first embodiment, it is however not possible to differentiate between the nucleic acid targets and to determine which nucleic acid target was indeed amplified.

As a mere, non-limiting example, it could be envisaged, in the case of SARS-CoV-2, to target the *N* gene, the E gene and the *ORF1ab* gene, using three pairs of nucleic acid primers, each pair being capable of hybridizing to a different target; and three nucleic acid probes, each being specific for one nucleic acid target and/or its amplification product but all three comprising the same detectable label or different detectable labels, as long as they have overlapping if not identical excitation wavelength ranges and/or overlapping if not identical emission wavelength ranges.

In a second embodiment where at least two nucleic acid targets are targeted (each being capable of hybridizing to a different nucleic acid target of the same genetic event and/or to its amplification product), each of the at least two nucleic acid probes is detected via a different, or dedicated, detection channel *(i.e.,* at different excitation range and emission wavelength ranges, or in other words, with non-overlapping excitation and emission wavelength ranges).

In this second embodiment, a multiple-channel system may be used for a multiplexed detection, such as, e.g., a two-channel, three-channel, four-channel, five-channel, or six-channel system; e.g., the three-channel digital PCR system "naica®" by Stilla Technologies.

In this second embodiment, each detection channel *(i.e.,* with a given excitation and/or emission wavelength range) can detect a single nucleic acid target. In the event that nucleic acid targets behave independently from each other - as explained hereinabove -, and by summing the number of positive events detected in all channels, the sensitivity of the assay can be multiplied by up to the number of independent nucleic acid targets, *i.e.,* the limit of detection (LOD) of the assay can be divided by up to the number of statistically independent nucleic acid targets. Moreover, as will be shown in detail below, if the assumption that the nucleic acids are fragmented, in a large volume, into corresponding nucleic acid targets prior to partitioning of the sample, is satisfied, then such feature also allows dividing the quantification precision error (also called "relative uncertainty") by the square root *of $k$, $k$* being the number of detection channels, *i.e.,* in this embodiment, the number of nucleic acid targets.

In this second embodiment, it is possible to differentiate between the nucleic acid targets, determine which nucleic acid target was indeed amplified and estimate the absolute quantification of each nucleic target in the analyzed volume.

As a mere, non-limiting example, it could be envisaged to target three nucleic acid targets, using three pairs of nucleic acid primers, each pair being capable of hybridizing to a different nucleic acid target; and three nucleic acid probes, each being specific for one nucleic acid target and each comprising a different detectable label. Using a six-channel system, it is even conceivable to detect and differentiate up to six different nucleic acid targets.

In this second embodiment, each detectable label used to detect a different nucleic acid target and/or its amplificon product allows in addition a quality control of the assay, based on the variability between the estimated absolute quantification of each nucleic acid target in the analyzed sample volume. In other words, if the nucleic acid targets behave independently from each other - as explained hereinabove -, the quantification value estimated for a given nucleic acid target should be similar to the quantification value estimated for another nucleic acid target in the analyzed sample. The closer the quantification values estimated for each nucleic acid target, the lower the uncertainty in the detection and the quantification of the nucleic acid target.

In a third embodiment where more than two multiple nucleic acid targets are targeted, it is conceivable to combine the first and second embodiments hereinabove, *i.e.,* detecting multiple independent nucleic acid targets and/or their amplification products in each of a plurality of detection channels, a given detection channel allowing for the detection of two or more nucleic acid targets which are different.

Both in the case where the different nucleic acid probes are attached or linked to the same detectable label and in the case where they are attached or linked to different detectable labels, it is possible to quantify the amount of nucleic acids using the different nucleic acid targets for increased sensitivity compared to the sensitivity that would obtained using only one nucleic acid target.

The advantages of targeting multiple targets to increase the sensitivity and decrease the uncertainty in any methods of analyzing a sample by digital PCR, regardless of the purpose of the test, will be apparent based on the following calculations.

Let us first consider an exemplary setup where two nucleic acid targets of a genetic event are targeted.

Let us define the following terms in Table 1:

**Table 1**

| Variable (unit) | Definition |
|---|---|
| C (copy/$\mu$L) | Estimated concentration of the nucleic acids representative of the genetic event in the subject's sample |
| d | Dilution factor from the subject's sample to the reaction mix (e.g., 10.5 $\mu$L of subject's sample mixed with reagents to a final volume of 25 $\mu$L prior to analysis, d = 25/10.5) |
| $C_1 = x_1 \times C$ (copy/$\mu$L) | Target$_1$ concentration for amplifiable Target$_1$ within the sample (with $x_1 <= 1$) |
| $C_2 = x_2 \times C$ (copy/$\mu$L) | Target$_2$ concentration for amplifiable Target$_2$ within the sample (with $x_2 <= 1$) |
| N | Total number of partitions analyzed |
| $p_{01}$ | Number of single positive partitions detected in channel 1 for Targeti |
| $p_{10}$ | Number of single positive partitions detected in channel 2 for Target$_2$ |
| $p_{11}$ | Number of double positive partitions detected (channels 1 and 2) |
| $p_1$ | Number of positive partitions detected in channel 1 ($p_1 = p_{01} + p_{11}$) |
| $p_2$ | Number of positive partitions detected in channel 2 ($p_2 = p_{10} + p_{11}$) |
| p | Number of positive partitions detected in at least one channel |
| v ($\mu$L) | Volume of a partitions (*e.g.*, in a Sapphire chip from the naica® System of Stilla Technologies, v = 0.00058592 $\mu$L) |

The concentration C may be calculated using any one of the following three equations:

(1):

$$C = d \times \frac{-\ln(1-\frac{p}{N})}{v(x_1+x_2)}$$

(2):

$$C = d \times \frac{-\ln(1-\frac{p_{01}+p_{10}+p_{11}}{N})}{v(x_1+x_2)}$$

(3):

$$C = d \times \frac{-\ln\left(1-\frac{p_1}{N}\right)-\ln\left(1-\frac{p_2}{N}\right)}{v(x_1+x_2)}$$

In the case where the two detectable labels are detected in the same detection channel *(i.e.,* at the same or overlapping excitation range and emission range), the only useful equation for calculating the concentration C is equation (1) above.

Besides, it is desirable that the fragmentation separating each nucleic acid target on a separate fragment has occurred either (1) in the subject's body before collecting the sample from said subject, and/or (2) after collecting the sample, in a sample volume that is significantly larger than the final volume analyzed by digital PCR. If this assumption of fragmentation in a large volume prior to partitioning of the sample is satisfied, then the LOD established in the analyzed volume can be extrapolated in the subject's body by simply applying all dilution factors, so that the presented methodology allows to divide by ($x_1 + x_2$) the LOD in the subject's body by using two nucleic acid targets compared to only one nucleic acid target.

If this assumption of fragmentation in a large volume prior to partitioning of the sample is satisfied, in both cases (*i.e.*, with two different detectable labels and two detection channels, or with two identical detectable labels and a single detection channel), the Limit Of Detection (LOD) with 95% confidence level in the analyzed volume will be, assuming the Limit Of Blank (LOB) is set at zero, in terms of number of partitions measured as positive for at least one channel:

$$LOD_{droplets}(95\%) = -\frac{\ln(0.05)}{x_1 + x_2}$$

and in terms of concentration in number of copies per µL in the subject's sample to be loaded in the reaction mix:

$$LOD(95\%) = -d\,\frac{\ln(0.05)}{(x_1 + x_2)\,N\,v}$$

In one embodiment, we can make the following approximation:

$$x_1 = x_2$$

(*i.e.,* both nucleic acid targets are equally amplifiable).

In one further embodiment, we can also make the following approximation:

$$x_1 = x_2 = 1$$

(*i.e.,* both nucleic acid targets are 100% amplifiable).

If the assumption of fragmentation in a large volume prior to partitioning of the sample is satisfied, and if it can be assumed that both nucleic acid targets are 100% amplifiable, we have a different equation allowing for a lower quantification uncertainty:

(4):

$$C = d \times \frac{-\ln(1 - \frac{p_1 + p_2}{2 \times N})}{v}$$

In another embodiment, we can make the following approximation:

$$x_1 + x_2 = 2$$

In yet another embodiment, we can make the following approximations:

$$\max(x_1, x_2) = 1$$

and

$$\frac{x_1}{x_2} = \frac{\ln(1 - \frac{p_1}{N})}{\ln(1 - \frac{p_2}{N})}$$

In this last specific embodiment, the skilled person will realize that with the associated assumptions, the calculations may be simplified.

To generalize beyond the two nucleic acid targets exemplified above, if the assumption of fragmentation large volume prior to partitioning of the sample is satisfied, the approach consisting in detecting a genetic event (such as, *e.g.*, the presence of a rare mutation, etc.) with *k* nucleic acid targets that are equally amplifiable and 100% amplifiable (with the assumption that these *k* different targets behave independently from each other during analysis, including during partitioning of the sample (*e.g.*, because they are each located on separate fragments as explained hereinabove), be they detected in *k* different detection channels or in the same and single detection channel, permits to divide the Limit Of Detection (LOD) of the genetic event by a factor *k (i.e.,* sensitivity of the digital PCR test is increased *k* times).

In such a situation, if the assumption of fragmentation in large volume prior to partitioning of the sample is satisfied, the LOD established in the analyzed volume can be extrapolated in the subject's body by simply applying all dilution factors, so that the presented methodology allows to divide by *k* the LOD in the subject's body by using *k* nucleic acid targets compared to only one nucleic acid target. Moreover, if this assumption of fragmentation in a large volume prior to partitioning of the sample is satisfied, then the presented methodology allows to divide by square root of *k* the quantification precision error (also called "relative uncertainty") by using k nucleic acid targets compared to only one nucleic acid target, by virtually multiplying by *k* the number of analyzed partitions.

If the assumption of fragmentation in a large volume prior to partitioning of the sample is not satisfied, then the quantification precision error is still decreased by the use of *k* targets but with a smaller decrease factor, thanks to the application of Poisson uncertainty to a *k* times higher concentration in the analyzed volume.

In order to provide a more general calculation for the case where *k* different detection channels are used to follow *k* different targets originating from nucleic acids representative of the same genetic event (and with the assumption that these *k* different targets behave independently from each other during analysis), we define the following variables in Table 2:

**Table 2**

| Variable (unit) | Definition |
|---|---|
| $k$ | Number of detection channels = number of independent nucleic acid targets targeted |
| $p_{(i)}$ | Number of positive partitions observed in the channel *i* for target *i* |
| $c_{cpd}$ | Average number of copies per partition |
| C (copy/$\mu$L) | Estimated concentration of nucleic acids representative of the genetic event in the subject's sample to be loaded in the reaction mix, when using all "k" targets |
| $C_1$ (copy/$\mu$L) | Estimated concentration of nucleic acids representative of the genetic event in the subject's sample to be loaded in the reaction mix, when using only one target, say target 1 |

The following general equation can be derived, assuming that all targets are equally amplifiable and 100% amplifiable:

$$c_{cpd} = - ln\left(1 - \frac{1}{k\,N}\sum_{i=1}^{k} p_{(i)}\right)$$

$$C = - \frac{d}{v}\, ln\left(1 - \frac{1}{k\,N}\sum_{i=1}^{k} p_{(i)}\right)$$

$$Uncertainty(C) = \frac{Uncertainty(C_1)}{\sqrt{k}}$$

Under the assumption of fragmentation in a large volume prior to partitioning of the sample, the Limit Of Detection with 95% confidence will be, assuming the Limit Of Blank is set at zero, in terms of number of positive partitions in at least one channel:

$$LOD_{partitions}(C\ ;\ 95\%) = -\frac{\ln(0.05)}{k}$$

$$LOD_{partitions}(C\ ;95\%) = \frac{LOD_{partitions}(C_1\ ;95\%)}{k}$$

and in terms of concentration (number of copies per µL) in the subject's sample to be loaded in the reaction mix:

$$LOD(C\ ;95\%) = -\,d\,\frac{\ln(0.05)}{k\,N\,v}$$

$$LOD(C\ ;95\%) = \frac{LOD(C_1\ ;95\%)}{k}$$

According to the invention, the method comprises a step of providing a conclusion regarding the presence of said genetic event based on whether fluorescence in at least one partition of the plurality of partitions is detected or not.

In one embodiment, the method comprises a step of concluding that the subject is positive for genetic event if fluorescence is observed in at least one partition of the plurality of partitions.

In one embodiment, the method comprises a step of concluding that at least one subject in the population of subjects is positive for said genetic event if fluorescence is observed in at least one partition of the plurality of partitions.

In one embodiment, the method comprises concluding that the genetic event is present if, for each nucleic acid probe, corresponding fluorescence is detected in at least one partition of the plurality of partitions.

In one embodiment, the subject is positive for said genetic event if fluorescence is observed in at least 1, 2, 3, 4, 5, 6, 7, 8, 9, 10 or more partitions of the plurality of partitions.

In one embodiment, at least one subject in the population of subjects is positive for said genetic event if fluorescence is observed in at least 1, 2, 3, 4, 5, 6, 7, 8, 9, 10 or more partitions of the plurality of partitions.

In one embodiment where each of the at least two nucleic acid probes - each capable of hybridizing to a different nucleic acid target and/or to its amplification product - is attached or linked to detectable labels having non-overlapping excitation wavelength ranges and/or non-overlapping emission wavelength ranges with respect to the other detectable label(s), the subject is positive for said genetic event if a fluorescence signal with a given emission wavelength range is detected in at least 1, 2, 3, 4, 5, 6, 7, 8, 9, 10 or more partitions of the plurality of partitions. In other words, the subject is positive for said genetic event if one of at least two nucleic acid targets is detected.

In one embodiment where each of the at least two nucleic acid probes - each capable of hybridizing to a different nucleic acid target and/or to its amplification product - is attached or linked to detectable labels having non-overlapping excitation wavelength ranges and/or non-overlapping emission wavelength ranges with respect to the other detectable label(s), the subject is positive for said genetic event if at least two fluorescence signals with a different emission wavelength range are detected in at least 1, 2, 3, 4, 5, 6, 7, 8, 9, 10 or more partitions of the plurality of partitions. In other words, the subject is positive for said genetic event if both or all of at least two nucleic acid targets are detected.

In one embodiment, the method according to the invention comprises a step of computing, for each nucleic acid target for which corresponding fluorescence is detected, a respective concentration. In this case, the method further concluding that a genetic event is present if fluorescence is detected in at least one partition of the plurality of partitions and if, for each nucleic acid target for which corresponding fluorescence is detected, the respective concentration is outside a corresponding predetermined concentration range.

This is particularly advantageous in the case where the genetic event is a chromosomal abnormality such as a monosomy, a trisomy, a tetrasomy, etc.

In one embodiment, the method according to the invention comprises concluding that the genetic event is present if fluorescence is not detected in the plurality of partitions. This is particularly advantageous in the case where the genetic event is a deletion, or an insertion or substitution in the sequence of the nucleic acid target.

In one embodiment, the method may comprise a step of dividing the population of subjects into two or more subgroups if fluorescence was observed in at least one partition of the plurality of partitions, and separately repeating the method of the invention with two or more pooled samples, each being obtained by pooling individual samples from each subject of one of the two or more subgroups.

In other words, the method may be reiterated on subgroups of the population of subjects, until all positive subjects are individually identified in the subgroups.

In one embodiment, the method may be run as follows:

1) Test a group of n subjects using the method described herein;

> *If the test is negative, mark all n subjects as negative and the protocol stops*
> *If the test is positive, form two subgroups of n/2 subjects, tagged S1 and S2*

2) Test the group S1 of n/2 subjects

> *If S1 is negative, mark all n/2 subjects of S1 as negative, and form two subgroups of n/4 subjects from subgroup S2, tagged S2-1 and S2-2;*
> *If S1 is positive, a) form two subgroups of n/4 subjects from subgroup S1, tagged S1-1 and S1-2; and b) test the group S2 of n/2 subjects*

3) Reiterate by dividing each positive subgroup in two, until a subgroup of 2 subjects is known to hold at least one virus holder, and test the two subjects.

In one embodiment, the at least two nucleic acid targets of the genetic event are RNA targets, and the method comprises a step of reverse-transcribing the RNA target, thereby obtaining at least two reverse-transcribed cDNA targets.

Means and methods for carrying out reverse transcription of RNA targets are well known to the one skilled in the art, using a reverse-transcriptase enzyme and a mix of 4 deoxyribonucleotides triphosphate (dNTPs), namely deoxyadenosine triphosphate (dATP), deoxycytidine triphosphate (dCTP), deoxyguanosine triphosphate (dGTP) and (deoxy)thymidine triphosphate (dTTP).

Reverse transcription typically comprises:

- a first-strand cDNA synthesis (thereby obtaining a double-stranded mixed RNA-cDNA library),
- optionally, an RNA templates removal (thereby obtaining a single-stranded cDNA library),
- a second-strand cDNA synthesis (thereby obtaining a double-stranded cDNA library), and
- optionally, a double-stranded cDNA library purification.

Methods for first-strand cDNA synthesis are well-known to the one skilled in the art. First-strand cDNA synthesis reactions can use a combination of sequence-specific primers, oligo(dT) or random primers.

Methods for RNA templates removal are well-known to the one skilled in the art. RNA template removal can be carried out, e.g., by incubating the double-stranded mixed RNA-cDNA library with RNase H.

RNAs reverse-transcription to generate a cDNA library can be carried out in a random manner, i.e., using random primers and thereby reverse-transcribing the whole or major part of the RNAs. Alternatively, RNAs reverse-transcription to generate a cDNA library can be carried out in a targeted manner, i.e., using specific primers and thereby creating a cDNA library of custom sequences only.

In one embodiment, the step of reverse-transcribing the RNA target is carried out after the step of pooling individual samples from each subject of the population of subjects, to obtain a pooled sample.

In one embodiment, the step of reverse-transcribing the RNA target is carried out after the step of partitioning the pooled sample into a plurality of partitions.

In the latter, the amplification mixture further comprises at least one reverse-transcriptase.

In one embodiment, the step of reverse-transcribing the RNA target is carried out before the step of partitioning the sample.

In one embodiment, the step of reverse-transcribing the RNA target is carried out before the step of pooling individual samples from each subject of the population of subjects, to obtain a pooled sample.

In the latter, the step of reverse-transcribing the RNA target optionally comprises labelling the at least one reverse-transcribed cDNA target from each subject of the population of subjects with a personalized index.

The term **"personalized index"** refers to a molecular pattern which can be used as a unique identifier, to uniquely identify the source (*i.e.,* the individual subject) of a nucleic acid target, such as a reverse-transcribed cDNA target, in a population of subjects. The principle is typically similar to that well known from one skilled in the art of single cell genomics and implemented to uniquely identify reads that originate from the same cell, although on a larger scale.

Personalized indexes may be of a variety of different formats, including labels, tags, probes, barcodes and the like.

In one embodiment, the personalized index is optically labeled. In one embodiment, the personalized index is non-optically labeled.

Optical labels include, but are not limited to, chromophores, fluorophores, quantum dots, styrene monomers, and combination thereof, which can be identified, *e.g.*, by their spectrum such as Raman spectrum or electromagnetic spectrum; and/or by their intensity of color.

Non-optical labels include, but are not limited to, biomolecular sequences, also termed "barcode", such as DNA, RNA and/or protein sequences, which can be identified, *e.g.*, by sequencing.

In one embodiment, the personalized index is a nucleic acid barcode. In one embodiment, each personalized index comprises a unique nucleic acid barcode, *i.e.,* a unique nucleic acid sequence.

In one embodiment where the at least one reverse-transcribed cDNA target from each subject of the population of subjects has been indexed with a personalized index, the method may further comprise a step of recovering partitions in which fluorescence was observed and identifying the personalized index of the at least one reverse-transcribed cDNA target, thereby identifying the subject(s) in the population of subjects being positive for the genetic event.

For example, the naica® System (Stilla Technologies) allows the recovery of droplets from the Sapphire Chips with a simple procedure that can be performed before or after amplification. Nucleic acids contained in the recovered partitions can be extracted using a standard chloroform protocol, and are suitable for downstream analysis using NGS, digital or real-time PCR, or gel electrophoresis.

In one embodiment, an endogenous nucleic acid target specific to the subject is targeted, as an internal control. In this embodiment, the partitions further comprise (in addition to the at least two pairs of nucleic acid primers capable of hybridizing to a different nucleic acid target of a same genetic event) one or more pairs of nucleic acid primers, each pair being capable of hybridizing to a subject's specific nucleic acid target, and one or more nucleic acid probes, each probe being capable of hybridizing to a subject's specific nucleic acid target and/or to its amplification product.

As used herein, the term **"endogenous nucleic acid target"** or **"subject's specific nucleic acid target"** refers to a nucleic acid sequence which is endogenously found in all the subjects of the population of subjects. Examples of such endogenous nucleic acid targets include, e.g., housekeeping genes.

The invention also relates to a non-invasive prenatal testing method. In this case, a sample is collected from a pregnant female subject carrying an embryo or a fetus. More precisely, the sample is collected in a non-invasive way. Then, the method disclosed above is performed on said collected sample. Finally, it is concluded that that said genetic event is present in the embryo or the fetus, *i.e.,* that the embryo or the fetus is positive for said genetic event, based on whether fluorescence is detected or not.

The present invention also relates to a system suitable for performing the method as described herein.

In one embodiment, the system may comprise one or several or all of the following:

- at least one individual container (also called "container"), each container being suitable for storing a sample from a subject;
- a subsystem and reactants for performing a digital PCR assay, said reactants including an amplification mixture comprising a polymerase, at least two pairs of nucleic acid primers, each capable of hybridizing to a respective nucleic acid targets of a genetic event, and at least two nucleic acid probes attached or linked to a detectable label; wherein each of the at least two pairs of nucleic acid primers is capable of hybridizing to a different nucleic acid target of the same genetic event, and wherein each of the at least two nucleic acid probes is capable of hybridizing to a different nucleic acid target of the same genetic event and/or to its amplification product; and
- a subsystem for detecting a fluorescence signal.

In one embodiment, the system further comprises at least one or several pooling container(s), each of the at least one or several pooling container(s) being suitable for storing a pooled sample comprising a pool of individual samples.

In one embodiment, the individual containers and pooling container(s) comprise an identification means, such as, *e.g.*, a barcode, and the system comprises a subsystem for identifying the individual containers and pooling container(s), such as, *e.g.*, a barcode reader.

In one embodiment, the system may further comprise a subsystem and reactants for extracting nucleic acids. In one embodiment, the system may further comprise a subsystem for detecting a fluorescence signal. In one embodiment, the system may further comprise a subsystem and reactants for performing a qPCR assay. In one embodiment, the system may further comprise a subsystem and reactants for artificially fragmenting nucleic acids.

In one embodiment, the system may further comprise pipetting means, for automatic or manual pipetting, transfer, mixing, and the like, of individual samples (*i.e.*, the samples) and/or pooled samples.

In one embodiment, the system comprises a processor for performing automatically the method of detecting a genetic event as described herein.

In one embodiment, the system is configured to pool at least a fraction of individual sample from each individual flask (*i.e.*, individual container) of the set of individual flasks or a subset thereof, into the at least one or several pooling flasks (*i.e.*, pooling containers).

In one embodiment, the system is further configured to extract nucleic acid from the pooled sample in the at least one or several pooling flasks.

In one embodiment, the system is further configured to partition the sample into a plurality of partitions, wherein said partitions comprise the amplification mixture.

In one embodiment, the system is further configured to perform a digital polymerase chain reaction (dPCR) on the plurality of partitions under conditions suitable to amplify, if present, the at least two nucleic acid targets of the genetic event.

In one embodiment, the system is further configured to detect fluorescence in the plurality of partitions, wherein a fluorescence signal in a given partition is indicative of amplification of at least one of the at least two nucleic acid targets of the genetic event in said given partition.

In one embodiment, the processor is configured to perform the step of detecting a genetic event based on fluorescence signals received from at least two microchambers containing a sample from the same subject.

This is advantageous. Indeed, the volume of the sample that is collected from a subject varies generally depending on the technique and/or the person that performs sampling. On the other hand, the volume of the microchambers of a microfluidic chip is predefined and cannot be changed. By detecting a genetic event based on fluorescence signals received from at least two microchambers containing a sample from the same subject, a situation where part of the sample is discarded is prevented.

Moreover, such feature entails an increase in the amount of nucleic acids subjected to the dPCR assay, which, in turn, increases the probability of detecting fluorescence in at least one partition.

In one embodiment, the system is further configured to reiterate the method by pooling at least a fraction of individual sample of each individual flask of a smaller subset of individual flasks into the at least one or several pooling flasks, depending on whether a fluorescence signal has been detected or not.

In one embodiment, the system is further configured to test individual samples individually, i.e., without pooling, if a fluorescence signal has been detected. In one embodiment, individual samples tested individually may be tested by qPCR.

## BRIEF DESCRIPTION OF THE DRAWINGS

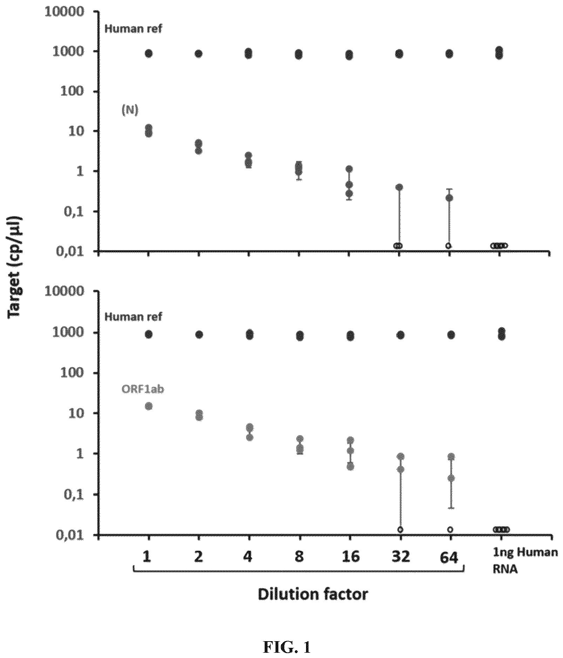

**Figure 1** is a set of two graphs showing the sensitivity of the Covid-19 digital PCR detection kit for SARS-CoV-2 detection. Serial dilutions of SARS-CoV-2 synthetic targets were assayed in triplicates in a background of 1 ng of human RNA. A total of 8 μL of positive controls was added to each 25 μL reaction. The vertical bars represent the theoretical 95 % Poisson confidence intervals for the pools of 3 replicates. Empty circles represent replicates where SARS-CoV-2 sequences were not detected. Upper graph: copies (cp) of SARS-CoV-2 N gene/μL of positive control; lower graph: copies (cp) of SARS-CoV-2 *ORF1ab* gene/μL of positive control.

Figure 2 is a flowchart illustrating the structure of the comparative study of Example 4.

Figure 3 is a histogram showing the distribution of Ct values for the E gene and *Orf* gene, as measured using individual reference RT-PCR with COBAS® 6800, for the 25 positive samples.

**Figure 4** is a set of two graphs showing the number of positive droplets for the N gene **(Figure 4A)** and *ORF1ab* gene **(Figure 4B)** per reaction *versus* the predicted equivalent Ct value of the *N* gene and the *ORF1ab* gene, respectively.

**Figure 5** is a workflow illustrating a complete, automatable process for sensitive pooled testing based on the method of the invention.

## EXAMPLES

The present invention is further illustrated by the following examples. The examples provided hereinafter relate to the detection of Sars-CoV-2 in a population of several subjects. Detection of Sars-CoV-2 is a reference example. However, the person skilled in the art understands that these teachings can be directly applied, without changes, to the detection of a genetic event according to the invention in a single subject, i.e., without pooling samples from a population of several subjects.

### Example 1

### Covid-19 digital PCR detection kit

The kit contains a specific ready-to-use system for detection of Sars-CoV-2 by one-step reverse transcription PCR using a digital PCR platform. A master mix contains mix for the specific amplification of virus RNA. The kit uses primers and probes targeting the SARS-CoV-2 *ORF1ab* and *N* genes, which are labeled with HEX and FAM fluorescence, respectively, and all other necessary detection reagents to prepare the reactions. After pressurization of the digital PCR system, the reaction solution is dispersed into 25000-30000 droplets in a well of the digital PCR chip. When a droplet contains the RNA of SARS-CoV-2, the corresponding primers and probes will pair with the nucleic acids, and reverse transcription (with a qScript XLT reverse transcriptase) followed by PCR amplification (with an AccuStart II Taq DNA polymerase) can occur and generate a fluorescence signal. In addition, the kit contains a set of primers and probes complementary to an internal human control (IC) and labeled with the CY5 fluorophore to identify possible PCR inhibition.

Assay procedure

The naica® System (Stilla Technologies) with a Sapphire Chip was used to partition the samples and carry out amplification, with the program described in **Table 3.**

**Table 3**

|   | Step | Temp (°C) | Time | Cycle |
|---|------|-----------|------|-------|
| **1** | Partition | 40°C | 12min | 1 |
| **2** | cDNA synthesis | 50°C | 10 min | 1 |
| **3** | Initial denaturation | 95°C | 1 min | 1 |
| **4** | Denaturation | 95°C | 10 sec | 45 |
| **5** | Annealing/Extension | 55°C | 30 sec | |
| **6** | Release | / | 33min | / |

Results analysis

Using a three-channel digital PCR system (naica® System, Stilla Technologies), the chips were moved after PCR to the naica® Prism3 device and results were analyzed using the "Crystal reader" software.
Results could be determined using the matrix described in Table 4 below.

**Table 4**

| Sample | | | Negative control | | Result |
|--------|--------|--------|------------------|--------------------|--------|
| **Channel 1 (FAM)** | **Channel 2 (HEX)** | **Channel 3 (CY5)** | **N gene (FAM)** | **ORF1ab gene (HEX)** | |
| **N gene** | **ORF1ab gene** | **Internal Control** | | | |
| + | + | + | - or ND | | Positive |
| + | + | < 10 | - or ND | | |
| If one target is + and one target is ND | | + | - or ND | | |
| If one target is + and one target is ND | | < 10 | - or ND | | Inconclusive |
| ND | ND | + | - | | Positive |

(continued)

| Sample | | | Negative control | | Result |
|---|---|---|---|---|---|
| Channel 1 (FAM) | Channel 2 (HEX) | Channel 3 (CY5) | *N* gene (FAM) | *ORF1ab* gene (HEX) | |
| *N* gene | *ORF1ab* gene | Internal Control | | | |
| ND | ND | + | If only one or if two targets, is ND (no +) | | Inconclusive |
| If one target is ND and one target is - | | + | If only one or if two targets, is ND (no +) | | |
| If one target is ND and one target is - | | + | - | | |
| - | - | + | - or ND | | Negative |
| - or ND | - or ND | < 10 | - or ND | | Inconclusive |
| If one target is + and one target is - | | < 10 or + | - or ND | | Negative |
| Any | Any | Any | + | | Invalid |
| *"+" means the number of positive droplets ≥ 3*<br>*"ND" means 1 or 2 positive droplets*<br>*"-" means 0 positive droplets*<br>*"Positive" means that SARS-Co V-2 was detected in the sample*<br>*"Negative" means that no SARS-CoV-2 was detected in the sample (because absent or below the detection limit).* | | | | | |

Assay Performance

*Limit of Detection (LoD) - Analytical Sensitivity*

A seven-fold serial dilution of a positive control containing synthetic RNA sequences targeted by the *ORF1ab* and *N* genes was assayed in triplicates. The limit of detection was defined as the lowest number of copies per $\mu L$ in the 10.5 $\mu L$ of RNA template of positive control detected in a sample in all replicates.
The limit of detection at a 95 % confidence level was 480 copies/mL in 10.5 $\mu L$ of input RNA.

*Inclusivity (analytical sensitivity)*

The complete genome sequence corresponding to the SARS-CoV-2 isolate Wuhan-Hu-1 with NCBI Reference Sequence NC_045512.2 was aligned to the Betacoronavirus sequences of the NCBI database using Megablast optimized for highly similar sequences. A total of 96 complete genome sequences of SARS-CoV-2 with > 99% percentage of identity were retrieved. Primers and probes targeting the *ORF1ab* and N genes were aligned to the complete genome sequences using MAFFT version 7 and displayed 100 % homology in 100 % of their length.

*Cross-reactivity (analytical specificity)*

*In silico* study: the amplicons generated by primers targeting the *ORF1ab* and *N* genes were aligned to the sequence of the SARS coronavirus Frankfurt 1 strain (GenBank: AY291315.1) and the closely related bat SARS-like coronavirus isolate bat-SL-CoVZC45 (GenBank: MG772933.1). The *ORFlab* detection model displayed 5 and 6 mismatches with the SARS coronavirus Frankfurt 1 strain and bat-SL-CoVZC45 sequence, respectively. The N detection model displayed 8 and 14 mismatches with the SARS coronavirus Frankfurt 1 strain and bat-SL-CoVZC45 sequence, respectively.
*In vitro* study: A total of 15 replicates containing 1 $\mu L$ of a human total RNA solution at 1 ng/$\mu L$ were analyzed using the kit. No false positive droplets were measured in all negative control replicates, so no false positives were detected for the *ORF1ab* or *N* gene.

The sequenced strains of the SARS-CoV-2 virus can be detected, and no crossover with human coronaviruses HCoV-229E, HCoV-HKU1, HCoV-OC43 or HCoV-NL63 was observed.

**Example 2**

**qPCR *versus* digital PCR**

Samples from 44 patients tested for Covid19 by qPCR at the institute hospital-universitaire (IHU) "Méditerranée Infection" (Marseille, France) were retrieved and tested by digital PCR using the Covid-19 digital PCR detection kit of Example 1.
Results initially obtained by qPCR are as shown in **Table 5** below.

**Table 5**

| Result | Number of patients |
|---|---|
| Negative | 14 |
| Positive | 12 |
| Inconclusive (Ct > 35) | 18 |

As can be seen, out of 44 patients, the cycle threshold (Ct) was above 35 cycles for 40 % of them (18/44), and their results were considered inconclusive.

Digital PCR to confirm negative qPCR results

The samples from the 14 patients determined to be negative by qPCR were tested by digital PCR using the Covid-19 digital PCR detection kit of Example 1. Results are shown in **Table 6** below.

**Table 6**

| Sample | *N* gene (FAM) | | *ORF1ab* gene (HEX) | | IC (CY5) | |
|---|---|---|---|---|---|---|
| | C (cp/uL) | Nbr Pos | C (cp/uL) | Nbr Pos | C (cp/uL) | Nbr Pos |
| #1 | 0 | 0 | 0 | 0 | 8.36 | 101 |
| #2 | 0 | 0 | 0 | 0 | 34.2 | 469 |
| #3 | 0 | 0 | 0 | 0 | 21 | 277 |
| #4 | 0 | 0 | 0 | 0 | 5.6 | 56 |
| #5 | 0 | 0 | 0 | 0 | 13.5 | 129 |
| #6 | 0 | 0 | 0 | 0 | 13.3 | 81 |
| #7 | 0 | 0 | 0.09 | 1 | 37 | 390 |
| #8 | 0 | 0 | 0 | 0 | 22.8 | 214 |
| #9 | 0 | 0 | 0 | 0 | 100.4 | 1024 |
| #10 | 0.1 | 1 | 0 | 0 | 37.5 | 384 |
| #11 | 0 | 0 | 0 | 0 | 42.8 | 486 |
| #12 | 0 | 0 | 0 | 0 | 11.6 | 142 |
| **#13** | **0.86** | **10** | **0.6** | **7** | **21.2** | **244** |
| #14 | 0 | 0 | 0.09 | 1 | 125.2 | 1337 |
| *C (cp/uL): copy number concentration* | | | | | | |

*Nbr Pos: number of positive droplets*

As can be seen, one patient (#13) tested negative by qPCR was clearly tested positive using the Covid-19 digital PCR detection kit and the matrix described in Table 2 of Example 1.

Digital PCR to confirm positive qPCR results

Similarly, the samples from the 12 patients determined to be positive by qPCR were tested by digital PCR using the Covid-19 digital PCR detection kit of Example 1.

All 12 patients were confirmed as positive.

Digital PCR to determine the status of inconclusive results

Finally, the samples from 18 patients with inconclusive results were tested by digital PCR using the Covid-19 digital PCR detection kit of Example 1. Results are shown in **Table 7** below.

**Table 7**

| Sample | *N* gene (FAM) | | *ORF1ab* gene (HEX) | | IC (CY5) | | Results | Ct value in qPCR |
|---|---|---|---|---|---|---|---|---|
| | C (cp/uL) | Nbr Pos | C (cp/uL) | Nbr Pos | C (cp/uL) | Nbr Pos | | |
| #27 | 0 | 0 | 0 | 0 | 11 | 132 | Negative | 37.32 |
| #28 | 0.7 | 8 | 0.44 | 5 | 165.4 | 1806 | Positive | 35.53 |
| #29 | 0.26 | 3 | 0.09 | 1 | 35.2 | 406 | Positive | 35.15 |
| #30 | 0.09 | 1 | 0.18 | 2 | 77.6 | 849 | Negative | 34.65 |
| #31 | 0 | 0 | 0 | 0 | 73.4 | 785 | Negative | 36.92 |
| #32 | 0.08 | 1 | 0 | 0 | 6.72 | 80 | Negative | 36.56 |
| #33 | 0.45 | 5 | 0.18 | 2 | 1047 | 8723 | Positive | 36.75 |
| #34 | 0.08 | 1 | 0.62 | 8 | 23.4 | 298 | Positive | 35.17 |
| #35 | 0.17 | 2 | 0.26 | 3 | 742.3 | 7057 | Positive | 34.37 |
| #36 | 0.31 | 4 | 0 | 0 | 30.2 | 387 | Positive | 37.43 |
| #37 | 0.16 | 2 | 0.39 | 5 | 11.9 | 151 | Positive | 34.95 |
| #38 | 0.16 | 2 | 0.16 | 2 | 0.78 | 10 | Negative | 38.17 |
| #39 | 2.47 | 34 | 1.96 | 27 | 25.8 | 352 | Positive | 33.78 |
| #40 | 0.22 | 3 | 0.44 | 6 | 46.9 | 636 | Positive | 36.35 |
| #41 | 0 | 0 | 0.16 | 2 | 55.5 | 703 | Negative | 36.66 |
| #42 | 0.53 | 7 | 0.68 | 9 | 48.9 | 637 | Positive | 34.3 |
| #43 | 0 | 0 | 0 | 0 | 160.9 | 2206 | Negative | 36.96 |
| #44 | 0.2 | 3 | 0.27 | 4 | 126.3 | 1801 | Positive | 36.47 |

Viral absence was confirmed in 7/18 patients and viral presence was confirmed in 11/18 patients.

A retrospective analysis of the Ct values obtained by qPCR (last column of **Table 7**) shows no correlation between the result and the Ct value, confirming the limits of qPCR.

Conclusion

In conclusion, the test by digital PCR of the present invention offers a substantial gain of sensibility as compared to the commonly used qPCR tests, and allows to investigate Covid19 serology of qPCR data > 35 Ct. A statistical analysis of these results allowed to determine a gain of sensitivity between about 32 and 64 times as compared to qPCR.

Moreover, a statistical analysis of the results obtained in this assay confirmed that each droplet in the digital PCR assay contained in average no more than a single nucleic acid target.

## Example 3

**Pooled testing**

An experimental model containing synthetic sequences targeted by the Covid-19 digital PCR detection kit of Example 1 was serially diluted and seven dilution points were assessed in triplicate. A total of 1 ng of human RNA was added to each replicate.

The results shown in **Figure 1** indicate a robust and specific detection of SARS-CoV-2 sequences down to 0.6 copies (cp)/$\mu$L of positive control (5 cp/25 $\mu$L reaction) of the *N* gene and down to 0.9 cp/$\mu$L of positive control (7 cp/25 $\mu$L reaction) of the *ORF1ab* gene in all tested samples (dilution factor 16).

Further dilutions showed an extremely sensitive but stochastic detection down to 0.25 cp/$\mu$L of positive control (2 cp/25 $\mu$L reaction) for both genes (dilution factor up to 64).

In parallel, a total of 15 controls containing only human RNA (1 ng/25 $\mu$L reaction) were tested as negative controls, and no false positives were observed.

These results indicate that the Covid-19 digital PCR detection kit of Example 1 is suitable for performing a "large pool" testing approach, by pooling and testing samples from a number of individuals in a single digital PCR assay, with a sensitive detection.

These results are in line with the results of Example 2, which allowed to determine a gain of sensitivity between about 32 and 64 times as compared to qPCR; hence it is conceivable that the Covid-19 digital PCR detection kit of Example 1 is suitable for testing a pooled sample comprising between 32 to 64 individual samples, in a single digital PCR assay, with at least the same sensitivity as qPCR.

This sensitivity and the precision can further be improved by using more targets on the viral RNA, since, as confirmed in Example 2, each droplet in the digital PCR assay contains in average no more than a single nucleic acid target. This is due to the fact that viral RNA is spontaneously cleaved during processing of the sample (in between 5 to 10 pieces), and that targets are chosen in such a way that they are sufficiently distant from each other to be on different pieces of the cleaved viral RNA.

Using more targets on the viral RNA, it is then conceivable, with a three-channel digital PCR system, such as the naica® System (Stilla Technologies), to dedicate the third channel to a viral target instead of an internal control (since a pooled sample of several individual samples will statistically always be positive for the internal control).

Additionally or alternatively, it is also conceivable to test more than one target in each channel; and/or to use a digital PCR system with more than 3 channels.

## Example 4

**Pooled testing**

Material and Methods

*Specimens collection, storage and pooling*

Nasopharyngeal swabs of 448 patients screened for Covid-19 in a Parisian hospital (France) between May 6th and May 26th, 2020 were included. All samples were collected in universal transport medium (UTM) (Virocult® or eSwabTM) and tested, within 15 hours maximum upon collection, for SARS-CoV-2 detection according to the local standards. Briefly, 400 $\mu$L of transport medium were tested by real-time Retro-Transcriptase Polymerase Chain Reaction (RT-PCR) (Cobas SARS-CoV-2 test, Roche). All the remaining volume of transport medium of all specimens were kept at +5°C.

No later than 24 hours after routine screening, all samples with a leftover UTM volume above 600 $\mu$L were systematically included in the grouped analysis. Thus, 125 $\mu$L of each included specimen were sampled and randomly mixed with seven others to generate a total of 56 pools of 8 specimens with a final volume of 1 mL per pool. Nucleic acids were extracted from each pool prior to viral titration by digital Retro-Transcriptase Polymerase Chain Reaction (RT-dPCR). The remaining volume of transport medium were stored at +5°C.

According to the current French ethical laws, samples used in the current study were only included after the completion of all analysis required for the patient's care.

*Detection of SARS-CoV-2 by routine individual RT-PCR testing*

All 448 specimens were analyzed individually on a Cobas® 6800 system (Roche, Switzerland) for Covid-19 screening using the Cobas® SARS-CoV-2 Test kit, which targets conserved regions for ORF-1a/b and E-gene, according to the manufacturer's recommendations. Briefly, for each specimen 400 μL of transport medium were mixed with 400 μL of Cobas® lysis buffer and loaded on the analyzer. During the run, extracts were eluted in 50 μL of which 27 μL were used in the RT-PCR amplification of *E* and *ORF1ab*. A sample was considered positive for routine screening of Covid-19 ("COBAS +") if either target had a Ct value below 40 PCR cycles.

All samples which had different results for RT-PCR and RT-dPCR were reassessed on the Cobas® 6800 system. In case of low remaining amounts of transport medium, the nasal swabs were vortexed again into the remaining transport medium diluted 1 to 10 with new transport medium.

*Nucleic acid extraction*

All nucleic acids extractions for droplet PCR assays were performed on a MagNA Pure LC 2.0 (Roche, Switzerland) using the MagNA Pure LC Total Nucleic Acid Isolation Kit (Roche, Switzerland) following manufacturer's instructions.

For all sample pools, the total pool volume of 1 mL was used for the extraction. For individual samples, 200 μL was diluted with 800 μL of buffer before extraction.

Nucleic acids were eluted from 1mL to 50 μL of the elution buffer provided with the kit and stored at +5°C for a maximum of 12 hours before analysis.

*Preparation of pools of 16 and 32 individuals*

After co-extraction of the 56 pools of 8 specimen (P8 extracts) and prior to viral titration by RT-dPCR, 28 pools of 16 individual samples (P16 pools) were obtained by mixing 15 μL of two P8 extracts and 14 pools of 32 (P32 pools) were obtained by mixing 10 μL of two P16 pools.

*Detection of SARS-CoV-2 by pooled RT-dPCR testing*

SARS-CoV-2 titration of the pooled samples by RT-dPCR was performed on the naica® system (Stilla Technologies, France) within the next three hours after extraction, using the COVID-19 Multiplex Digital PCR Detection Kit (Stilla Technologies, France/Apexbio, China). This one-step reverse-transcription and triplex PCR kit amplifies one sequence in the *N* gene, one sequence in the *ORF1ab* region of SARS-CoV-2 and an endogenous internal control (IC) to assess the quality of the sample and extraction. These sequences are targeted by three TaqMan probes respectively labelled with a FAM, HEX and Cy®5 fluorophore.

As recommended by the kit manufacturer, the PCR mix for a single reaction contained 12.5 μL of dPCR MasterMix 1, 1 μL of dPCR Mix 2, 1 μL of COVID-19 Assay and 10.5 μL of either, P8, P16, P32, positive control, negative control or individual extract. 25 μL of this PCR mix were loaded in the inlet ports of the Sapphire chips (Stilla Technologies, France) and the chips were placed in the naica® Geode (Stilla Technologies, France) for droplets generation, reverse transcription and PCR amplification following the kit manufacturer's instructions.

After amplification, the chips were transferred to the naica® Prism3 (Stilla technologies, France) for fluorescence reading in the three detection channels and data were analyzed with Crystal Miner Software (Stilla Technologies, France) following the kit manufacturer's instructions.

*Individual confirmatory testing for SARS-CoV-2 by RT-PCR and RT-dPCR*

When needed, a confirmatory RT-PCR test was performed on individual samples. 200 μL of transport medium from samples diluted into 800 μL of phosphate buffered saline (PBS) were extracted individually on the MagNAPure LC 2.0 instrument following the same protocol as before. Confirmatory RT-PCR testing was perform using the RealStar® SARS-CoV-2 RT-PCR Kit (Altona Diagnostics, Germany) on an ABI 7500 thermocycler (ThermoFisher Scientific, United-States). This kit targets all lineage B-betacoronaviruses by amplifying a sequence of the *E* gene and a sequence of the *S* gene specific to SARS-CoV-2 as well as a heterologous internal control. In this study, a sample is considered positive to SARS-CoV-2 if the Ct value of either target is below 40 PCR cycles. Confirmatory testing by RT-dPCR was performed following the same method as described above.

*Summary of the method of the comparative study*

Overall, all samples from the cohort of 448 patients are tested for SARS-CoV-2 by

i) individual RT-PCR (Cobas® 6800),
ii) RT-dPCR in 56 pools of 8,
iii) RT-dPCR in 28 pools of 16, and
iv) RT-dPCR in 14 pools of 32;

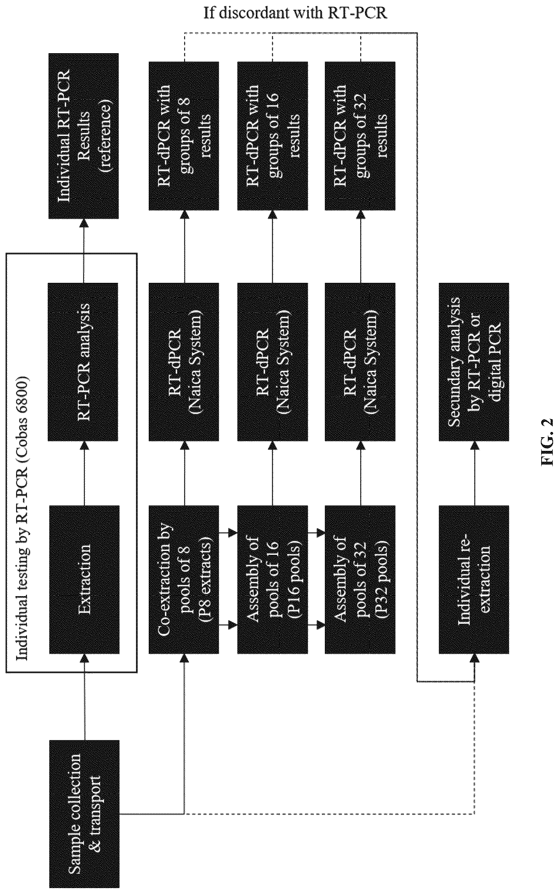

and results are compared between all four test protocols. In case of discordance between the results of individual RT-PCR testing and pooled testing in RT-dPCR, samples were re-analyzed individually by RT-dPCR and RT-PCR (Altona).
The protocol is illustrated in **Figure 2.**

*LoB/LoD evaluation for SARS-CoV-2 detection using RT-dPCR*

The Limit of Blank (LoB) and Limit of Dection (LoD) wer evaluated for SARS-CoV-2 detection using the pooling approach used in the study on a cohort of 256 pre-epidemic nasal swab samples (negative control samples) that were collected between December 1st 2019 and January 31st, 2020 and for which transport medium was stored at -20 °C.
Specimens were randomly pooled into 32 pools of 8 negative controls which were co-extracted and analyzed by RT-dPCR using the same protocol described above. The results for all 32 pools are given in **Table 8.**

**Table 8**

| Sample ID | Number of droplets | Positive droplets (*N+ORF1ab*) | Positive droplets (N) | Positive droplets (*ORF1ab*) | IC (cp/ μL) |
|---|---|---|---|---|---|
| Pool 1 | 19460 | 0 | 0 | 0 | 31430 |
| Pool 2 | 20641 | 0 | 0 | 0 | 10985 |
| Pool 3 | 21449 | 0 | 0 | 0 | 4054 |
| Pool 4 | 20983 | 0 | 0 | 0 | 15143 |
| Pool 5 | 19881 | 1 | 1 | 0 | 7401 |
| Pool 6 | 20609 | 0 | 0 | 0 | 8783 |
| Pool 7 | 19044 | 0 | 0 | 0 | 6249 |
| Pool 8 | 20787 | 0 | 0 | 0 | 8759 |
| Pool 9 | 21470 | 0 | 0 | 0 | 10271 |
| Pool 10 | 18952 | 0 | 0 | 0 | 17578 |
| Pool 11 | 23424 | 0 | 0 | 0 | 8222 |
| Pool 12 | 22748 | 0 | 0 | 0 | 9875 |
| Pool 13 | 23747 | 0 | 0 | 0 | 10275 |
| Pool 14 | 24886 | 0 | 0 | 0 | 4266 |
| Pool 15 | 24079 | 0 | 0 | 0 | 10264 |
| Pool 16 | 24718 | 0 | 0 | 0 | 13219 |
| Pool 17 | 24228 | 0 | 0 | 0 | 10186 |
| Pool 18 | 23742 | 0 | 0 | 0 | 8431 |
| Pool 19 | 23880 | 0 | 0 | 0 | 7628 |
| Pool 20 | 23613 | 0 | 0 | 0 | 3111 |
| Pool 21 | 25723 | 0 | 0 | 0 | 8987 |
| Pool 22 | 25522 | 0 | 0 | 0 | 7411 |
| Pool 23 | 24781 | 0 | 0 | 0 | 6894 |

(continued)

| Sample ID | Number of droplets | Positive droplets (*N+ORF1ab*) | Positive droplets (N) | Positive droplets (*ORF1ab*) | IC (cp/μL) |
|---|---|---|---|---|---|
| Pool 24 | 7074 | 0 | 0 | 0 | 5884 |
| Pool 25 | 27003 | 0 | 0 | 0 | 8911 |
| Pool 26 | 27138 | 0 | 0 | 0 | 5552 |
| Pool 27 | 24425 | 0 | 0 | 0 | 7886 |
| Pool 28 | 27017 | 0 | 0 | 0 | 7484 |
| Pool 29 | 26722 | 0 | 0 | 0 | 9963 |
| Pool 30 | 26088 | 0 | 0 | 0 | 2492 |
| Pool 31 | 26747 | 0 | 0 | 0 | 5703 |
| Pool 32 | 26728 | 0 | 0 | 0 | 5825 |

The LoB at 95 % confidence level for the *N* target and the *ORF1ab* target is determined to be of 2 and 0 positive droplets respectively. The LoD at 95 % confidence level for each target is of 0.46 copies/μL and 0.22 copies/μL respectively.

Consequently, a threshold of a least 3 positive droplets in aggregate between both the *N* target and *ORF1ab* target is used to classify a sample as positive to SARS-CoV-2 by RT-dPCR in this study.

Results

*Cohort description from routine RT-PCR testing*

Using routine RT-PCR testing, 25 samples were identified as positive out of the 448 samples tested, corresponding to an average test positivity rate of 5.5 %. The positivity rate decreased during the study, in correlation with the decrease in the disease prevalence observed during the month of May in France. The positivity rate was of 8.5 % for the first 224 samples and of 2.5 % for the last 224 samples.

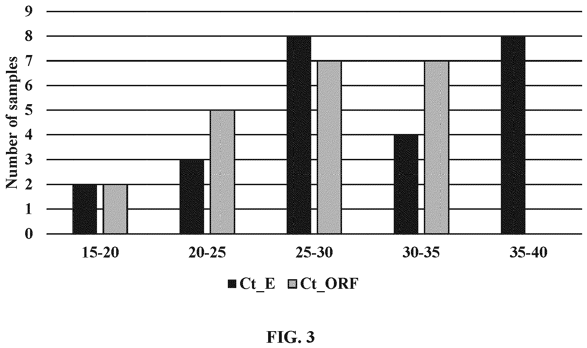

The average Ct value was of 30.0 and 27.3 for the *E* gene and *ORF* gene respectively, with minimum values of 16.5 and 16.3 and maximum values of 38.7 and 34.8 (**Fig. 3**).

*Results from pooled RT-dPCR testing*

**Table 9** presents the overall results for the detection of SARS-CoV-2 by RT-dPCR for the pooled extracts (P8) and the subsequent pools of 16 (P16) and 32 (P32).

**Table 9**

| Number of "COBAS +" samples in pool | Results for P8 extracts | | | Results for P16 pools | | | Results for P32 pools | | |
|---|---|---|---|---|---|---|---|---|---|
| | Total | dPCR - | dPCR + | Total | dPCR - | dPCR + | Total | dPCR - | dPCR + |
| 0 | 35 | 32 | 3 | 12 | 11 | 1 | 2 | 2 | 0 |
| 1 | 18 | 1 | 17 | 10 | 2 | 8 | 6 | 0 | 6 |
| 2 | 2 | 0 | 2 | 4 | 0 | 4 | 3 | 0 | 3 |
| 3 | 1 | 0 | 1 | 1 | 0 | 1 | 1 | 0 | 1 |
| 4 | 0 | 0 | 0 | 1 | 0 | 1 | 1 | 0 | 1 |
| 5 | 0 | 0 | 0 | 0 | 0 | 0 | 0 | 0 | 0 |
| 6 | 0 | 0 | 0 | 0 | 0 | 0 | 1 | 0 | 1 |
| Total | 56 | 33 | 23 | 28 | 12 | 16 | 14 | 2 | 12 |

Because sample pooling was performed randomly as samples came in the laboratory for routing RT-PCR testing, the pools contain variable numbers of RT-PCR positive samples ("COBAS +" samples).

Given the positivity rate of RT-PCR at the time of the study, the majority of P8 extracts had no "COBAS +" samples (35 out of 56) and amongst the 23 pools that contained at least one "COBAS +" sample, 18 had one single "COBAS +" sample.

For the largest pool size of 32 samples, the distribution is opposite. Only two P32 pools have no "COBAS +" samples. Amongst the twelve P32 pools with at least one "COBAS +" sample, only 6 had one single "COBAS +" sample and the maximum number of "COBAS +" samples was 6.

These observed distributions are in good agreement with theoretical distributions predicted assuming a test positivity rate of 5.5 % and random distribution.

*Detailed results for RT-dPCR in groups of 8*

The results for SARS-CoV-2 detection by RT-dPCR in groups of 8 are in concordance with the reference individual RT-PCR testing for 56 pools (corresponding for 448 samples), out of which 33 are pools with all samples negative by individual RT-PCR testing ("COBAS - pools") and 23 are pools that contain at least one sample positive by individual RT-PCR testing ("COBAS + pools").

4 pools had discording results:

- 3 "COBAS - pools" were found positive by RT-dPCR ("COBAS -/dPCR +" discordances): P8_20, P8_28 and P8_39;
- 1 "COBAS + pool" was found negative by RT-dPCR ("COBAS +/dPCR-" discordance): P8_02.

Out of the eight individual samples with Ct > 35 for the *E* gene, six ended up to be the only positive sample in a P8 extract. These six P8 extracts all tested positive by RT-dPCR, indicating that pooled testing by RT-dPCR is capable of detecting samples with late Ct values and low viral RNA concentrations. The highest detected Ct values for the *E* gene and *ORF1ab* is of [38.7; *not detected*].

The Ct values for the sample associated with the "COBAS +/dPCR -" discordance were of 34 and 32.3 for the *E* gene and *ORF1ab* respectively. This sample is referred to as "Sample _25659" for later discussion. "COBAS -/dPCR +" discordances are discussed further below.

**Table 10** shows a confusion matrix for P8 extracts.

**Table 10**

| | Expected negatives (RT-PCR) | Expected positives (RT-PCR) | Total |
|---|---|---|---|
| **Negatives in digital PCR** | 32 | 1 | 33 |
| **Positives in digital PCR** | 3 | 20 | 23 |

(continued)

| | Expected negatives (RT-PCR) | Expected positives (RT-PCR) | Total |
|---|---|---|---|
| Total | 35 | 21 | 56 |

*Detailed results for RT-dPCR in groups of 16*

The results for SARS-CoV-2 detection by RT-dPCR in pools of 16 are in concordance with individual RT-PCR testing for 25 pools (corresponding for 400 samples), out of which 11 were "COBAS - pools" and 14 were "COBAS + pools".
3 pools had discording results:

- 1 "COBAS -/dPCR +" discordance: P16_14;
- 2 "COBAS +/dPCR -" discordances: P16_13 and P16_28.

Out of the 8 individual samples with Ct > 35 for the *E* gene, 5 ended up to be the only positive sample in a P16 pool. two out of these five P16 pools tested negative by RT-dPCR and were responsible for the 2 "COBAS +/dPCR -" discordances.
The *E* gene and *OFR1ab* Ct values for these two samples were of [36.7; *not detected*] and [36.3; 34.2], while the highest detected Ct values were [38.3; *not detected*].
**Table 11** shows a confusion matrix for P16 pools.

**Table 11**

| | Expected negatives (RT-PCR) | Expected positives (RT-PCR) | Total |
|---|---|---|---|
| Negatives in digital PCR | 11 | 2 | 13 |
| Positives in digital PCR | 1 | 14 | 15 |
| Total | 12 | 16 | 28 |

*Detailed results for RT-dPCR in groups of 32*

The results for SARS-CoV-2 detection by RT-dPCR in pools of 32 are in concordance with individual RT-PCR testing for all 14 pools (corresponding for 448 samples).
Out of the eight individual samples with Ct > 35 for the *E* gene, three ended up to be the only positive sample in a P32 pool. All such three P32 pools tested positive by RT-dPCR. The highest detected Ct values for the *E* gene and *ORF1ab* was of [36.7; *not detected*].
Surprisingly, the two samples that were associated with the 2 "COBAS +/dPCR -" discordances (with Ct values of [36.7; *not detected*] and [36.3; 34.2] respectively) were both amongst the three P32 pools discussed above. Consequently, these two samples were successfully detected by RT-dPCR in pools of 32 (P32_07 & P32_14) but not detected in the pools of 16 (P16_13 & P16_28).
In one case (P32_07), the pool of 32 was a combination of two P16 pools with opposite discordances: P16_13 (COBAS +/dPCR -) and P16_14 (COBAS -/dPCR +). It is likely that the RNA templates from the P16_14 pool were those detected in the P32_07 pool.
In the case of P32_14, the number of positive droplets observed in RT-dPCR was right above the threshold of three positive droplets ($N=2$; $ORF1ab=1$) while for the corresponding pool of 16 (P16_28), the number of positive droplets was just below with 2 droplets ($N=2$; $ORF1ab=1$). In this case, statistical variations due to sampling error could explain the observation.
However, the dataset for P32 pools was not large enough to draw reliable conclusions regarding the sensitivity of pooled testing with pools of 32, in particular because most pools contained more than 1 positive sample.
**Table 12** shows a confusion matrix for P32 pools.

**Table 12**

| | Expected negatives (RT-PCR) | Expected positives (RT-PCR) | Total |
|---|---|---|---|
| Negatives in digital PCR | 2 | 0 | 2 |
| Positives in digital PCR | 0 | 12 | 12 |

(continued)

|  | Expected negatives (RT-PCR) | Expected positives (RT-PCR) | Total |
|---|---|---|---|
| Total | 2 | 12 | 14 |

*Further investigation of "COBAS -/dPCR + " discordances*

Three P8 extracts (P8_20, P8_28 and P8_39) and one P16 pool (P16_14) were tested positive by digital PCR while containing only samples that tested negatives by individual RT-PCR with the Cobas® 6800 system. P16_14 originates from a combination of P8_27 (COBAS -/dPCR -) and P8_28.

To further investigate these "COBAS -/dPCR +" discordances, confirmatory testing RT-dPCR was performed on all individual samples from the three P8 extracts. For each pool, one sample tested positive by individual RT-dPCR, with measured concentrations of viral RNA ranging from 128 copies per reaction to 2 copies per reaction for the *N* gene, and from 106 to 1 copies for the *ORF1ab* gene.

The 3 samples identified were retested on the Cobas® 6800 system and by confirmatory individual RT-PCR using the Altona kit. 2 samples tested positive using the Altona kit and only the sample with the highest measured concentration by RT-dPCR (Sample 56075) tested positive on the Cobas® 6800 with a high Ct value of 36.7 for the *E* gene while the *ORF* gene was not detected.

The sample that tested negative with the Altona kit had borderline levels of positive droplets in RT-dPCR (*N*=2; *ORF1ab*=1) and is likely to be below the level of detection of the Altona kit and Cobas® 6800 system.

Based on these results, the "COBAS -/dPCR +" samples are interpreted to be true positive samples that were not correctly assessed by the initial reference RT-PCR test on the Cobas® 6800 system.

**Table 13** shows the results of the individual reassessment by both RT-dPCR and RT-PCR (Altona and Cobas®) for the samples from the 3 "COBAS -/dPCR +" discordant pools. NT: not tested; ND: not detected.

**Table 13**

| Sample ID | COBAS reference | P8 extract number | dPCR+/dPCR- | N cp/rnx | ORF1ab cp/rnx | IC cp/rnx | Altona E gene | Altona S gene | COBAS N | COBAS OFR |
|---|---|---|---|---|---|---|---|---|---|---|
| 51996 | COBAS- | 20 | dPCR- | 1 | 0 | 7 502 | NT | NT | NT | NT |
| 52019 | COBAS- | 20 | dPCR- | 0 | 0 | 25 944 | NT | NT | NT | NT |
| 52031 | COBAS- | 20 | dPCR- | 0 | 0 | 68 983 | NT | NT | NT | NT |
| 52035 | COBAS- | 20 | dPCR- | 0 | 0 | 28 215 | NT | NT | NT | NT |
| 52042 | COBAS- | 20 | dPCR+ | 16 | 19 | 89 747 | 33 | 31.9 | ND | ND |
| 52047 | COBAS- | 20 | dPCR- | 0 | 0 | 122 825 | NT | NT | NT | NT |
| 52060 | COBAS- | 20 | dPCR- | 0 | 0 | 17 237 | NT | NT | NT | NT |
| 52062 | COBAS- | 20 | dPCR- | 0 | 0 | 105 711 | NT | NT | NT | NT |
| 56075 | COBAS- | 28 | dPCR+ | 128 | 106 | 69 268 | 29 | 28.4 | 36.7 | ND |
| 56077 | COBAS- | 28 | dPCR- | 0 | 0 | 34 656 | NT | NT | NT | NT |
| 56083 | COBAS- | 28 | dPCR- | 0 | 0 | 71 410 | NT | NT | NT | NT |
| 56191 | COBAS- | 28 | dPCR- | 0 | 0 | 22 471 | NT | NT | NT | NT |
| 56211 | COBAS- | 28 | dPCR- | 0 | 0 | 21 185 | NT | NT | NT | NT |
| 56275 | COBAS- | 28 | dPCR- | 0 | 0 | 22 771 | NT | NT | NT | NT |
| 56303 | COBAS- | 28 | dPCR- | 0 | 0 | 56 797 | NT | NT | NT | NT |
| 56307 | COBAS- | 28 | dPCR- | 0 | 0 | 59 159 | NT | NT | NT | NT |
| 60241 | COBAS- | 39 | dPCR- | 0 | 0 | 46 988 | NT | NT | NT | NT |
| 60281 | COBAS- | 39 | dPCR- | 1 | 0 | 73 581 | NT | NT | NT | NT |
| 60310 | COBAS- | 39 | dPCR- | 0 | 0 | 11 420 | NT | NT | NT | NT |
| 60334 | COBAS- | 39 | dPCR- | 0 | 0 | 32 663 | NT | NT | NT | NT |
| 60345 | COBAS- | 39 | dPCR- | 0 | 0 | 81 004 | NT | NT | NT | NT |
| 60362 | COBAS- | 39 | dPCR- | 0 | 0 | 36 943 | NT | NT | NT | NT |
| 60389 | COBAS- | 39 | dPCR- | 0 | 0 | 825 | NT | NT | NT | NT |
| 60401 | COBAS- | 39 | dPCR+ | 2 | 1 | 43 651 | ND | ND | ND | ND |

EP 4 136 258 B1

*Further investigation of the sample Cobas +/dPCR -*

One pool of 8 (P8_02) tested negative by digital PCR while it contained one sample (*Sample _25659*) which tested positive by reference RT-PCR with Ct value of 34 and 32.3 for the *E* gene and *ORF1ab* respectively. These Ct values are not particularly high and pools containing samples with significantly higher Ct values, up to 38, were reliable detected in this study.

Sample _25659 was subsequently re-extracted individually and retested by individual digital PCR testing, confirmatory RT-PCR using the Altona kit and Cobas® routine screening method.

Sample _25659 was found to be borderline negative by individual digital PCR (*N*=2; *ORF1ab*=0) but Ct values of 37.3 and 34.9 were found for *E* and *ORF* respectively in the second Cobas® assessment.

*Correlation between digital PCR measurements and Ct values*

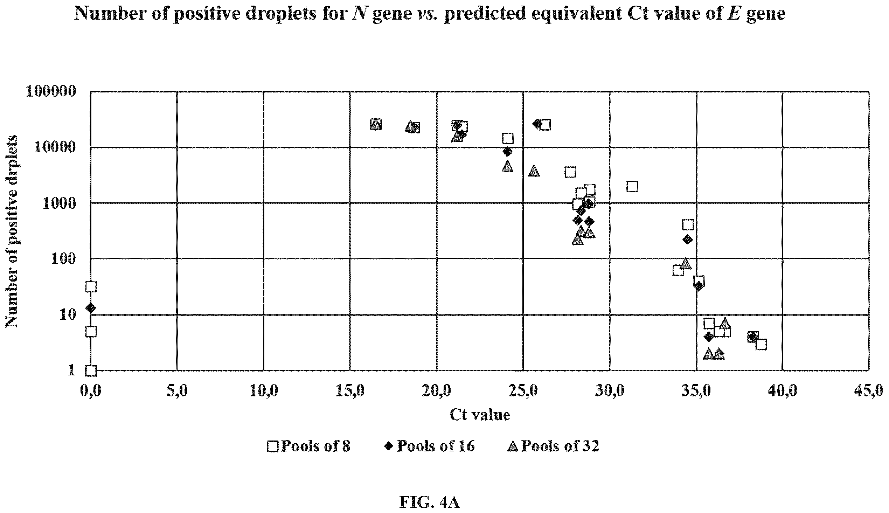

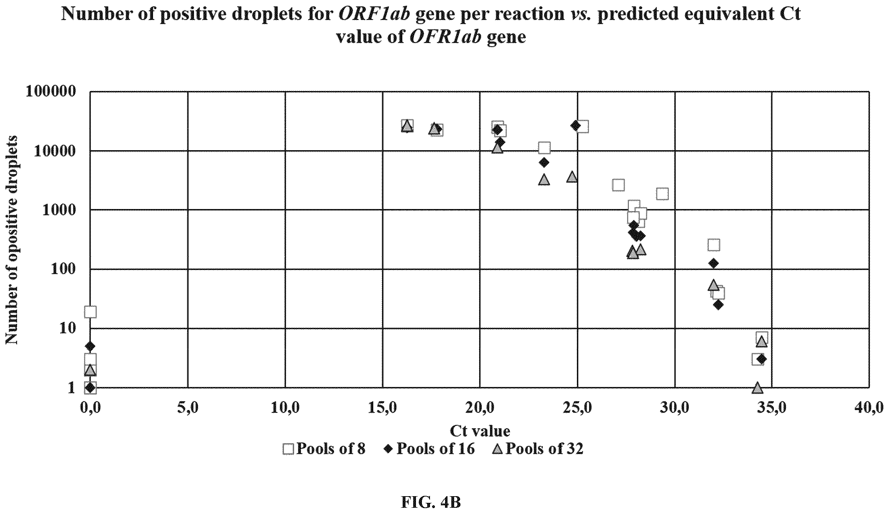

It is noteworthy that a good correlation is observed between the number of positive droplets observed in RT-dPCR and the log-average Ct value of the individual samples contained in the group, as seen in **Fig. 4A** (for the *N* gene) and **Fig. 4B** (for the *ORF1ab* gene).

Discussion

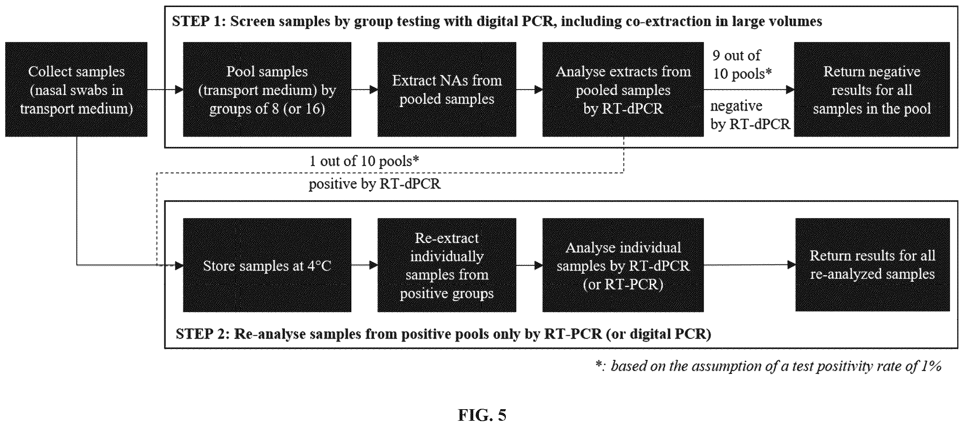

Based on these results, we assessed the sensitivity of group testing combined with digital PCR, for the 3 different group sizes investigated. To do so, we suggest a practical protocol in which the first step of group screening is performed by RT-dPCR as disclosed herein, and in which the second step of individual re-testing of positive groups is performed using RT-dPCR as well (**Fig. 5**).

In this sensitivity analysis, the 3 samples associated with the COBAS -/dPCR + discordances are considered to be true positive samples containing the SARS-CoV-2 virus responsible of Covid-19. The sample associated with the COBAS +/dPCR - is also considered a true positive sample. Further investigation by sequencing is underway on these four samples in an effort to rigorously assess the nature of the nucleic acids responsible for the discording results.

Based on these assumptions, this study suggests that group testing by RT-dPCR has a better sensitivity than individual RT-PCR testing for pools of 8 samples. In the cohort of 448 samples, a total of 23 groups of 8 tested positive and included 27 true positive samples. Only 25 samples were identified as positive using reference individual RT-PCR testing. This corresponds to a +8% improvement in sensitivity.

In the case of pools of 16, the data indicates that grouped testing by RT-dPCR has similar sensitivity to individual RT-PCR testing. A total of 15 groups of 16 tested positive and included 26 true positive samples. Compared with the 25 true positive samples identified by RT-PCR, this corresponds to +4% improvement in sensitivity.

Testing in pools of 32 by RT-dPCR has 100% concordance with the reference RT-PCR testing. Re-testing positive groups by RT-dPCR would have likely led to better sensitivity than RT-PCR.

We also discuss an alternative and more cost-effective group testing protocol in which the re-testing step would be performed using RT-PCR with Cobas or Altona kits. In these protocols, the sensitivity becomes dependent on the RT-PCR kit used. With an Altona kit, our data suggest that testing with pools of 8 and 16 would still have a better or similar sensitivity.

Overall, our data indicates that Covid-19 pooled testing by digital PCR, followed by re-testing by RT-PCR or digital PCR, has better to similar sensitivity than individual RT-PCR testing, with large group sizes of up to 16, and possibly 32 samples. By comparison, multiple studies have found that Covid-19 group testing by RT-PCR had lower sensitivity than individual testing, the loss of sensitivity increasing with the group size.

Here, the gain in sensitivity of the method disclosed herein is due to a concentration effect due to performing the pooling prior extraction and performing the extraction step from a large volume of 1 mL of pooled transport medium, and to the intrinsic superior sensitivity of digital PCR compared to RT-PCR.

The loss of sensitivity is one of the main reasons why group testing has not been widely adopted for Covid-19 testing, whilst research groups have advocated for its implementation as a solution to the worldwide unmet demand for tests and reagent shortage.

This study suggests that the high-sensitivity of group testing by digital PCR makes the approach viable for large-scale, low-cost patient screening with minimum reagent consumption.

**Claims**

**1.** A method of detecting a genetic event, the method comprising the steps of:

a) partitioning a sample from a subject into a plurality of partitions, wherein said partitions comprise an amplification mixture comprising:

 i. a polymerase;
 ii. at least two pairs of nucleic acid primers, each pair of nucleic acid primers being capable of hybridizing to a respective nucleic acid target of the genetic event; and
 iii. at least two nucleic acid probes, each nucleic acid probe being capable of hybridizing to a respective nucleic acid target of the genetic event, and being attached or linked to a different detectable fluorescent label, the different detectable fluorescent labels having non-overlapping excitation wavelength ranges and/or non-overlapping emission wavelength ranges;

b) performing a digital polymerase chain reaction (dPCR) assay on the plurality of partitions under conditions suitable to amplify, if present, each nucleic acid target of the genetic event;
c) providing a conclusion regarding the presence of said genetic event based on whether fluorescence in at least one partition of the plurality of partitions is detected or not, wherein fluorescence in a given partition is indicative of amplification of at least one nucleic acid target of the genetic event in said given partition;
wherein the at least two nucleic acid targets are located, upon spontaneous and/or artificial fragmentation of nucleic acids in the sample, on separate fragments of said nucleic acids, thereby statistically behaving independently from each other during partitioning of the sample into a plurality of partitions, and therefore behaving independently from each other in the dPCR assay,
the genetic event being a monosomy, a trisomy or a tetrasomy.

2. The method according to claim **1**, further comprising a step of computing, for each nucleic acid target for which corresponding fluorescence is detected, a respective concentration, and wherein step c) comprises concluding that said genetic event is present if fluorescence is detected in at least one partition of the plurality of partitions and if, for each nucleic acid target for which corresponding fluorescence is detected, the respective concentration is outside a corresponding predetermined concentration range.

3. The method according to claim **1**, wherein step c) comprises concluding that the genetic event is present if fluorescence is not detected in the plurality of partitions.

4. The method according to any one of claims **1** to **3**, wherein each nucleic acid target of the genetic event is an RNA target, and the method comprises a step of reverse-transcribing the RNA target, thereby obtaining at least one reverse-transcribed cDNA target.

5. The method according to claim **4**, wherein the step of reverse-transcribing is carried out after step a).

6. The method according to any one of claims **1** to **5**, wherein spontaneous and/or artificial fragmentation of the nucleic acids occurs in the sample, before step a) of partitioning the sample into a plurality of partitions.

7. The method according to any one of claims **1** to **6**, comprising, before step a), a step of artificially fragmentating nucleic acids in the sample, in conditions suitable to have the at least two nucleic acid targets located on separate fragments of said nucleic acids, thereby statistically behaving independently from each other during the partitioning of the sample into a plurality of partitions at step a).

8. The method according to claim **7**, wherein artificially fragmentating nucleic acids is carried out by contacting the sample with at least one sequence-specific endonuclease.

9. The method according to claim **8**, wherein the at least one sequence-specific endonuclease is selected from sequence-specific endonucleases capable of cleaving the nucleic acids between the at least two nucleic acid targets, thereby having the at least two nucleic acid targets located on separate fragments of said nucleic acids upon artificial fragmentation and statistically behaving independently from each other during the partitioning of the sample into a plurality of partitions at step a).

10. The method according to any one of claims **1** to **9**, wherein the genetic event is at least one of: Down syndrome 21, Edwards syndrome 18, Patau syndrome 13, trisomy 9, tetrasomy 9p, Warkany syndrome 2, cat eye syndrome, trisomy 22, trisomy 16, 1q21.1 deletion syndrome, 1q21.1 duplication syndrome, TAR syndrome, 1p36 deletion syndrome, Wolf-Hirschhorn syndrome, cri du chat syndrome, chromosome 5q deletion syndrome, Williams syndrome, Jacobsen syndrome, Miller-Dieker syndrome, Smith-Magenis syndrome, DiGeorge syndrome, 22q11.2 distal deletion syndrome, 22q13 deletion syndrome, Angelman syndrome, Prader-Willi syndrome, distal 18q-, proximal 18q-, Turner syndrome, Klinefelter syndrome, XXYY syndrome, XXXY syndrome, 49XXXYY syndrome, 49XXXXY syndrome, triple X syndrome, tetrasomy X, 49XXXXX, Jacobs syndrome, 48XYYY, 49XYYYY, 45X/46XY, 46XX/46XY.

11. A non-invasive prenatal testing method, comprising performing the steps of the method according to any one of claims **1** to **10**, the sample being a blood sample from a pregnant female subject carrying an embryo or a fetus, and wherein step c) is a step of providing a conclusion regarding the presence of said genetic event in the fetus of the embryo based on whether fluorescence in at least one partition of the plurality of partitions is detected or not.

12. A system for performing the method according to any one of claims **1** to **10**, or the non-invasive prenatal testing method of claim **11**, the system comprising:

- at least one container suitable for storing a sample from a subject;
- a subsystem and reactants for performing a digital PCR assay, said reactants including an amplification mixture comprising:

i. a polymerase;
ii. at least two pairs of nucleic acid primers, each pair of nucleic acid primers being capable of hybridizing to a respective nucleic acid target of the genetic event; and
iii. at least two nucleic acid probes, each nucleic acid probe being capable of hybridizing to a respective nucleic acid target of the genetic event, and being attached or linked to a different detectable fluorescent label, the different detectable fluorescent labels having non-overlapping excitation wavelength ranges and/or non-overlapping emission wavelength ranges;

- a subsystem for detecting a fluorescence signal;
the system optionally further comprising at least one of:
- a subsystem and reactants for extracting nucleic acids;
- a subsystem and reactants for artificially fragmenting nucleic acids;
- pipetting means.

**Patentansprüche**

1. Verfahren zum Nachweis eines genetischen Ereignisses, wobei das Verfahren die folgenden Schritte umfasst:

a) Aufteilen einer Probe von einem Patienten in eine Vielzahl von Partitionen, wobei die Partitionen ein Amplifikationsgemisch umfassen, umfassend:

i. eine Polymerase;
ii. wenigstens zwei Paare von Nucleinsäureprimern, wobei jedes Paar von Nucleinsäureprimern an ein jeweiliges Nucleinsäureziel des genetischen Ereignisses hybridisieren kann; und
iii. wenigstens zwei Nucleinsäuresonden, wobei jede Nucleinsäuresonde an ein jeweiliges Nucleinsäureziel des genetischen Ereignisses hybridisieren kann und an einen unterschiedlichen nachweisbaren Fluoreszenzmarker gebunden oder damit verknüpft ist, wobei die unterschiedlichen nachweisbaren Fluoreszenzmarker nichtüberlappende Anregungswellenlängenbereiche und/oder nichtüberlappende Emissionswellenlängenbereiche aufweisen;

b) Durchführen eines digitalen Polymerase-Kettenreaktion(dPCR)-Assays mit der Vielzahl von Partitionen unter Bedingungen, die geeignet sind, jedes Nucleinsäureziel des genetischen Ereignisses, falls vorhanden, zu amplifizieren;
c) Ziehen einer Schlussfolgerung bezüglich der Anwesenheit des genetischen Ereignisses auf der Basis, ob in wenigstens einer Partition der Vielzahl von Partitionen eine Fluoreszenz nachgewiesen ist oder nicht, wobei eine Fluoreszenz in einer gegebenen Partition darauf hinweist, dass wenigstens ein Nucleinsäureziel des genetischen Ereignisses in der jeweiligen Partition amplifiziert ist;
wobei sich die wenigstens zwei Nucleinsäureziele nach spontaner und/oder künstlicher Fragmentierung von Nucleinsäuren in der Probe auf getrennten Fragmenten der Nucleinsäuren befinden, wobei sie sich während der Aufteilung der Probe in eine Vielzahl von Partitionen statistisch unabhängig voneinander verhalten und sich daher im dPCR-Assay unabhängig voneinander verhalten, wobei das genetische Ereignis eine Monosomie, Trisomie oder Tetrasomie ist.

2. Verfahren gemäß Anspruch 1, weiterhin umfassend einen Schritt des Berechnens einer jeweiligen Konzentration für jedes Nucleinsäureziel, für das eine entsprechende Fluoreszenz nachgewiesen wurde, wobei Schritt c) die Schlussfolgerung umfasst, dass das genetische Ereignis vorhanden ist, wenn in wenigstens einer Partition der Vielzahl von Partitionen eine Fluoreszenz nachgewiesen wurde und wenn für jedes Nucleinsäureziel, für das eine entsprechende Fluoreszenz nachgewiesen ist, die jeweilige Konzentration außerhalb eines entsprechenden vorbestimmten Konzentrationsbereichs liegt.

3. Verfahren gemäß Anspruch 1, wobei Schritt c) die Schlussfolgerung umfasst, dass das genetische Ereignis vorhanden ist, wenn in der Vielzahl von Partitionen keine Fluoreszenz nachgewiesen ist.

4. Verfahren gemäß einem der Ansprüche 1 bis 3, wobei jedes Nucleinsäureziel des genetischen Ereignisses ein RNA-Ziel ist und das Verfahren einen Schritt des reversen Transkribierens des RNA-Ziels umfasst, wobei man wenigstens ein revers transkribiertes cDNA-Ziel erhält.

5. Verfahren gemäß Anspruch 4, wobei der Schritt des reversen Transkribierens nach Schritt a) durchgeführt wird.

6. Verfahren gemäß einem der Ansprüche 1 bis 5, wobei vor Schritt a) des Aufteilens der Probe in eine Vielzahl von Partitionen eine spontane und/oder künstliche Fragmentierung der Nucleinsäuren in der Probe erfolgt.

7. Verfahren gemäß einem der Ansprüche 1 bis 6, umfassend: vor Schritt a) einen Schritt des künstlichen Fragmentierens von Nucleinsäuren in der Probe unter geeigneten Bedingungen, um die wenigstens zwei Nucleinsäureziele auf getrennten Fragmenten der Nucleinsäuren vorzufinden, wobei sie sich während der Aufteilung der Probe in eine Vielzahl von Partitionen in Schritt a) statistisch unabhängig voneinander verhalten.

8. Verfahren gemäß Anspruch 7, wobei das künstliche Fragmentieren von Nucleinsäuren durchgeführt wird, indem man die Probe mit wenigstens einer sequenzspezifischen Endonuclease in Kontakt bringt.

9. Verfahren gemäß Anspruch 8, wobei die wenigstens eine sequenzspezifische Endonuclease aus sequenzspezifischen Endonucleasen, die die Nucleinsäuren zwischen den wenigstens zwei Nucleinsäurezielen spalten können, ausgewählt ist, wobei sich die wenigstens zwei Nucleinsäureziele nach der künstlichen Fragmentierung auf getrennten Fragmenten der Nucleinsäuren vorfinden und sie sich während der Aufteilung der Probe in eine Vielzahl von Partitionen in Schritt a) statistisch unabhängig voneinander verhalten.

10. Verfahren gemäß einem der Ansprüche 1 bis 9, wobei das genetische Ereignis wenigstens eines der folgenden ist: Down-Syndrom 21, Edwards-Syndrom 18, Patau-Syndrom 13, Trisomie 9, Tetrasomie 9p, Warkany-Syndrom 2, Katzenaugen-Syndrom, Trisomie 22, Trisomie 16, 1q21.1-Deletionssyndrom, 1q21.1-Duplikationssyndrom, TAR-Syndrom, 1p36-Deletionssyndrom, Wolf-Hirschhorn-Syndrom, Katzenschrei-Syndrom, Chromosom-5q-Deletionssyndrom, Williams-Syndrom, Jacobsen-Syndrom, Miller-Dieker-Syndrom, Smith-Magenis-Syndrom, DiGeorge-Syndrom, 22q11.2-Distal-Deletionssyndrom, 22q13-Deletionssyndrom, Angelman-Syndrom, Prader-Willi-Syndrom, Distal-18q-, Proximal-18q-, Turner-Syndrom, Klinefelter-Syndrom, XXYY-Syndrom, XXXY-Syndrom, 49XXXYY-Syndrom, 49XXXXY-Syndrom, Triple-X-Syndrom, Tetrasomie X, 49XXXXX, Jacobs-Syndrom, 48XYYY, 49XYYYY, 45X/46XY, 46XX/46XY.

11. Nichtinvasives pränatales Testverfahren, umfassend das Durchführen der Schritte des Verfahrens gemäß einem der Ansprüche 1 bis 10, wobei die Probe eine Blutprobe von einer Schwangeren ist, die ein Embryo oder einen Fötus trägt, und wobei Schritt c) ein Schritt des Ziehens einer Schlussfolgerung bezüglich der Anwesenheit des genetischen Ereignisses in dem Fötus oder dem Embryo auf der Basis, ob in wenigstens einer Partition der Vielzahl von Partitionen eine Fluoreszenz nachgewiesen ist oder nicht, ist.

12. System zum Durchführen des Verfahrens gemäß einem der Ansprüche 1 bis 10 oder des nichtinvasiven pränatalen Testverfahrens gemäß Anspruch 11, wobei das System umfasst:

- wenigstens einen Behälter, der geeignet ist, eine Probe von einem Patienten aufzubewahren;
- ein Teilsystem und Reaktanten zur Durchführung eines digitalen PCR-Assays, wobei die Reaktanten ein Amplifikationsgemisch umfassen, umfassend:

i. eine Polymerase;
ii. wenigstens zwei Paare von Nucleinsäureprimern, wobei jedes Paar von Nucleinsäureprimern an ein jeweiliges Nucleinsäureziel des genetischen Ereignisses hybridisieren kann; und
iii. wenigstens zwei Nucleinsäuresonden, wobei jede Nucleinsäuresonde an ein jeweiliges Nucleinsäureziel des genetischen Ereignisses hybridisieren kann und an einen unterschiedlichen nachweisbaren Fluoreszenzmarker gebunden oder damit verknüpft ist, wobei die unterschiedlichen nachweisbaren Fluoreszenzmarker nichtüberlappende Anregungswellenlängenbereiche und/oder nichtüberlappende Emissionswellenlängenbereiche aufweisen;

- ein Teilsystem zum Nachweisen eines Fluoreszenzsignals; wobei das System gegebenenfalls weiterhin wenigstens eines der folgenden umfasst:
- ein Teilsystem und Reaktanten zum Extrahieren von Nucleinsäuren;
- ein Teilsystem und Reaktanten zur künstlichen Fragmentierung von Nucleinsäuren;
- eine Pipettiereinrichtung.

## Revendications

1. Procédé de détection d'un événement génétique, le procédé comprenant les étapes de :

a) partitionnement d'un échantillon d'un sujet en une pluralité de partitions, lesdites partitions comprenant un mélange d'amplification comportant :

i. une polymérase ;
ii. au moins deux paires d'amorces d'acide nucléique, chaque paire d'amorces d'acide nucléique étant capable de s'hybrider à une cible d'acide nucléique respective de l'événement génétique ; et
iii. au moins deux sondes d'acide nucléique, chaque sonde d'acide nucléique étant capable de s'hybrider à une cible d'acide nucléique respective de l'événement génétique, et étant attachée ou liée à un marqueur fluorescent détectable différent, les différents marqueurs fluorescents détectables présentant des plages de longueurs d'onde d'excitation disjointes et/ou des plages de longueurs d'onde d'émission disjointes ;

b) mise en oeuvre d'un test de réaction en chaîne par polymérase digitale (dPCR) sur la pluralité de partitions dans des conditions appropriées pour amplifier, si elle est présente, chaque cible d'acide nucléique de l'événement génétique ;
c) fourniture d'une conclusion concernant la présence dudit événement génétique en fonction du fait que la fluorescence dans au moins une partition de la pluralité de partitions est détectée ou non, dans lequel la fluorescence dans une partition donnée est indicative de l'amplification d'au moins une cible d'acide nucléique de l'événement génétique dans ladite partition donnée ;

dans lequel les au moins deux cibles d'acide nucléique sont situées, lors de la fragmentation spontanée et/ou artificielle des acides nucléiques dans l'échantillon, sur des fragments séparés desdits acides nucléiques, se comportant ainsi statistiquement indépendamment l'une de l'autre pendant la partition de l'échantillon en une pluralité de partitions, et se comportant donc indépendamment l'une de l'autre dans le test dPCR, l'événement génétique étant une monosomie, une trisomie ou une tétrasomie.

2. Procédé selon la revendication 1, comprenant, en outre, une étape de calcul, pour chaque cible d'acide nucléique pour laquelle une fluorescence correspondante est détectée, d'une concentration respective, et dans lequel l'étape c) comprend la conclusion que ledit événement génétique est présent si une fluorescence est détectée dans au moins une partition de la pluralité de partitions et si, pour chaque cible d'acide nucléique pour laquelle une fluorescence correspondante est détectée, la concentration respective est en dehors d'une plage de concentration prédéterminée correspondante.

3. Procédé selon la revendication 1, dans lequel l'étape c) comprend la conclusion que l'événement génétique est présent si la fluorescence n'est pas détectée dans la pluralité de partitions.

4. Procédé selon l'une quelconque des revendications 1 à 3, dans lequel chaque cible d'acide nucléique de l'événement génétique est une cible d'ARN, et le procédé comprend une étape de transcription inverse de la cible d'ARN, obtenant ainsi au moins une cible d'ADNc transcrite inversement.

5. Procédé selon la revendication 4, dans lequel l'étape de transcription inverse est réalisée après l'étape a).

6. Procédé selon l'une quelconque des revendications 1 à 5, dans lequel une fragmentation spontanée et/ou artificielle des acides nucléiques se produit dans l'échantillon, avant l'étape a) de partitionnement de l'échantillon en une pluralité de partitions.

7. Procédé selon l'une quelconque des revendications 1 à 6, comprenant, avant l'étape a), une étape de fragmentation artificielle d'acides nucléiques dans l'échantillon, dans des conditions appropriées pour que les au moins deux cibles d'acide nucléique soient situées sur des fragments distincts desdits acides nucléiques, se comportant ainsi statistiquement indépendamment l'une de l'autre lors du partitionnement de l'échantillon en une pluralité de partitions à l'étape a).

8. Procédé selon la revendication 7, dans lequel la fragmentation artificielle des acides nucléiques est réalisée en mettant en contact l'échantillon avec au moins une endonucléase spécifique de séquence.

9. Procédé selon la revendication 8, dans lequel l'au moins une endonucléase spécifique de séquence est choisie parmi les endonucléases spécifiques de séquence capables de cliver les acides nucléiques entre les au moins deux cibles d'acide nucléique, de sorte que les au moins deux cibles d'acide nucléique soient situées sur des fragments séparés desdits acides nucléiques lors de la fragmentation artificielle et se comportent statistiquement indépendamment l'une de l'autre pendant le partitionnement de l'échantillon en une pluralité de partitions à l'étape a).

10. Procédé selon l'une quelconque des revendications 1 à 9, dans lequel l'événement génétique est au moins l'un parmi : syndrome de Down 21, syndrome d'Edwards 18, syndrome de Patau 13, trisomie 9, tétrasomie 9p, syndrome de Warkany 2, syndrome de l'œil de chat, trisomie 22, trisomie 16, syndrome de délétion lq21.1, syndrome de duplication lq21.1, syndrome TAR, syndrome de délétion lp36, syndrome de Wolf-Hirschhom, syndrome du cri du chat, syndrome de délétion du chromosome 5q, syndrome de Williams, syndrome de Jacobsen, syndrome de Miller-Dieker, syndrome de Smith-Magenis, syndrome de DiGeorge, syndrome de délétion distale 22qll.2, syndrome de délétion 22ql3, syndrome d'Angelman, syndrome de Prader-Willi, syndrome 18q- distal, syndrome 18q- proximal, syndrome de Turner, syndrome de Klinefelter, syndrome XXYY, syndrome XXXY, syndrome 49XXXYY syndrome, syndrome 49XXXXY, syndrome triple X, tétrasomie X, 49XXXXX, syndrome de Jacobs, 48XYYY, 49XYYYY, 45X/46XY, 46XX/46XY.

11. Procédé de test prénatal non invasif, comprenant la mise en oeuvre des étapes du procédé selon l'une quelconque des revendications 1 à 10, l'échantillon étant un échantillon de sang provenant d'un sujet féminin enceinte portant un embryon ou un foetus,
et dans lequel l'étape c) est une étape de fourniture d'une conclusion concernant la présence dudit événement génétique dans le foetus de l'embryon en fonction du fait que la fluorescence dans au moins une partition de la pluralité de partitions est détectée ou non.

12. Système pour la mise en oeuvre du procédé selon l'une quelconque des revendications 1 à 10, ou le procédé de test prénatal non invasif selon la revendication 11, le système comprenant :

- au moins un récipient adapté pour le stockage d'un échantillon provenant d'un sujet ;
- un sous-système et des réactifs pour la mise en oeuvre d'un test PCR digitale, lesdits réactifs comprenant un mélange d'amplification comportant :

i. une polymérase ;
ii. au moins deux paires d'amorces d'acide nucléique, chaque paire d'amorces d'acide nucléique étant capable de s'hybrider à une cible d'acide nucléique respective de l'événement génétique ; et
iii. au moins deux sondes d'acide nucléique, chaque sonde d'acide nucléique étant capable de s'hybrider à une cible d'acide nucléique respective de l'événement génétique, et étant attachée ou liée à un marqueur fluorescent détectable différent, les différents marqueurs fluorescents détectables ayant des plages de longueurs d'onde d'excitation disjointes et/ou des plages de longueurs d'onde d'émission disjointes ;

- un sous-système de détection d'un signal de fluorescence ;

le système comprenant optionnellement, en outre, au moins l'un parmi :
- un sous-système et des réactifs pour extraire des acides nucléiques ;
- un sous-système et des réactifs pour fragmenter artificiellement des acides nucléiques ;
- des moyens de pipetage.

**FIG. 1**

**FIG. 2**

**FIG. 3**

Number of positive droplets for *N* gene *vs.* predicted equivalent Ct value of *E* gene

□ Pools of 8    ◆ Pools of 16    ▲ Pools of 32

FIG. 4A

**Number of positive droplets for *ORF1ab* gene per reaction *vs.* predicted equivalent Ct value of *OFR1ab* gene**

FIG. 4B

EP 4 136 258 B1

**STEP 1: Screen samples by group testing with digital PCR, including co-extraction in large volumes**

Collect samples (nasal swabs in transport medium) → Pool samples (transport medium) by groups of 8 (or 16) → Extract NAs from pooled samples → Analyse extracts from pooled samples by RT-dPCR

9 out of 10 pools* negative by RT-dPCR → Return negative results for all samples in the pool

1 out of 10 pools* positive by RT-dPCR

**STEP 2: Re-analyse samples from positive pools only by RT-PCR (or digital PCR)**

Store samples at 4°C → Re-extract individually samples from positive groups → Analyse individual samples by RT-dPCR (or RT-PCR) → Return results for all re-analyzed samples

*: based on the assumption of a test positivity rate of 1%

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2240576 A


**Non-patent literature cited in the description**

- **TAN CHIANRU et al.** A multiplex droplet digital PCR assay for non-invasive prenatal testing of fetal aneuploidies. *ANALYST,* 08 January 2019, vol. 144 (7), 2239-2247